# EUROPEAN PATENT APPLICATION

(11) **EP 2 505 200 A1**
(43) Date of publication of application: **03.10.2012**
(21) Application number: 12174490.8
(22) Date of filing: 18.03.2005
(51) Int. Cl.: A61K 31/445, C07D 401/00, C07D 401/04

(54) **Methods of using and compositions comprising immunomodulatory compounds for the treatment and management of scleroderma**

(30) Priority: 22.03.2004 US 554923 P
(62) Divisional of application: 05732239.8
(71) Applicant: CELGENE CORPORATION, Summit, NJ 07901 (US)
(72) Inventor: Zeldis, Jerome B., Princeton, NJ 08540 (US); Hariri, Robert J., Bernardsville, NJ 07924 (US)
(74) Representative: Jones Day

(57) **Abstract**

The invention relates to methods of treating, preventing, correcting and/or managing skin diseases or disorders characterized by overgrowths of the epidermis, keratoses, scleroderma, cutaneous vasculitis, acne or wrinkles are disclosed. Specific embodiments encompass the administration of an immunomodulatory compound, or a pharmaceutically acceptable salt, solvate, hydrate, stereoisomer, clathrate, or prodrug thereof, alone or in combination with a second active agent. Specific second active ingredients are capable of affecting or inhibiting cell growth or proliferation, removing or improving acne scars, or reducing or correcting wrinkle lines. Pharmaceutical compositions, single unit dosage forms, and kits suitable for use in methods of the invention are also disclosed.

## Description

### 1. FIELD OF THE INVENTION

This invention relates to methods of treating, preventing and managing keratoses, scleroderma, cutaneous vasculitis, skin diseases or disorders characterized by overgrowths of the epidermis, acne or wrinkles, which comprise the administration of an immunomodulatory compound alone or in combination with known therapeutics. The invention also relates to pharmaceutical compositions and dosing regimens.

### 2. BACKGROUND OF THE INVENTION

### 2.1. TYPES OF SKIN DISEASES

### 2.1.1 KERATOSIS

Keratosis refers to a diverse group of skin diseases or disorders characterized by lesions on the epidermis marked by the presence of circumscribed overgrowths of the horny layer of the skin. Specific types of keratosis include, but are not limited to, actinic keratosis, seborrheic keratosis, keratoacanthoma, keratosis follicularis (Darier disease), inverted follicular keratosis, palmoplantar keratoderma (PPK, also known as keratosis palmaris et plantaris), keratosis pilaris, and stucco keratosis. Stedman, Medical Dictionary, 1990, 25th ed., 823.

Actinic keratosis is also known as senile keratosis, keratosis senilis, verruca senilis, plana senilis, solar keratosis, or keratoderma. Stedman, Medical Dictionary, 1990, 25th ed., 823; and Clay J. Cockerell, J. Am. Acad. Dermatol. 2000, 42:S11-7. Actinic keratosis is characterized by scaly, red, epidermal lesions that can develop into malignant non-melanoma skin cancer. Steven R. Feldman et al., J. Am. Acad. Dermatol. 1999, 40:43-7. Lesions typically have an erythematous base covered by scale (hyperkeratosis). Steven R. Feldman et al., J. Am. Acad. Dermatol. 1999, 40:43-7. Actinic keratoses are characterized by disordered epidermal differentiation. The epidermis in actinic keratoses may be atrophic or of normal thickness, or it may be of increased thickness with proliferation of abnormally differentiated epithelium extending into the papillary dermis. With time, actinic keratoses may develop into invasive cutaneous horns or skin cancers. Richard G. Glogau, J. Am. Acad. Dermatol. 2000, 42:S23-4.

Actinic keratosis can be caused by carcinogens and UV light exposure, and occurs chiefly in sun-exposed areas of the face, ears, forearms, and dorsum of hands. Lynn A. Drake et al., J. Am Acad. Dermatol. 1995, 32:95-8. However, it may occur on any area that is chronically or repeatedly exposed to the sun, such as the back, chest, and legs. The specific effect of ultraviolet radiation on the tumor suppressor gene p53 suggests a cause-effect relationship between ultraviolet radiation and the earliest mutations seen in actinic keratoses. David J. Leffell, J. Am. Acad. Dermatol. 2000, 42:S18-22. Moreover, a genetic predisposition for actinic keratoses has been reported in fair, redheaded, and blonde patients who bum frequently and tan poorly. Stuart J. Salasche, J. Am. Acad. Dermatol. 2000, 42:S4-7. No sex predilection has been found, except that sun exposure tends to be higher in males due to more exposure from outside activities. One of the most important factors in determining the expression of actinic keratoses is age. Epidemiologic studies indicate that actinic keratoses increase in prevalence with advancing age. *Id.* at S5. The age of occurrence is related to the skin type and amount of sun damage. Thus, while actinic keratosis can occur in patients aged 20-30 years, it is more common in patients aged 30-60 years.

Seborrheic keratosis, also known as seborrheic wart or senile wart, is a benign tumor commonly found in advanced and middle-aged persons. Merck Index, 1999 (17^{th} ed.), 841. A familial predisposition is apparent with an autosomal inheritance. Seborrheic keratosis also may be a consequence of inflammatory dermatoses or malignancies. It is typically characterized by a raised papular lesion of variable color from light to dark brown. Seborrheic keratosis may be smooth or wartlike with visible pitting. Common sites include the face, trunk, and extremities. *Id.* A variant known as dermatosis papulosa nigra occurs over the forehead and malar regions of individuals with black skin, occurs at an earlier age, and is smaller and smoother than variants in persons with fairer skin. Irritated seborrheic keratosis is one subtype, characterized by a predominance of basal cells with whorling sheets of squamous cells. All seborrheic keratoses are characterized by varying degrees of acanthosis, papillomatosis, hyperkeratosis, and hyperpigmentation of the basal cells. *Id.*

Another type of keratosis is inverted follicular keratosis, which is believed to be an inflammatory variant of seborrheic keratosis. It is commonly found on the faces and sun-exposed areas of elderly patients. Typically, this lesion is located on the upper eyelid. The lesion tends to be single and present as a papule or nodule. The hallmarks of this lesion are the plentiful squamous eddies. A papillomatous and acanthotic appearance is found. The margins are discrete and lack the inflammatory component found in keratoacanthoma (eMedicine Website, benign skin lesions).

Another type of keratosis is keratoacanthoma, which is characterized by round, firm, usually flesh-colored nodules with sharply sloping borders and a characteristic center crater containing keratinous material. Merck Index, 1999 (17th ed.), 842. Its rapid growth rate is frequently adequate to allow the clinician to distinguish keratoacanthoma from malignancy because it grows much more quickly than carcinoma. *Id.* It tends to occur in males more often than in females, with a male-to-female ratio of 3-4:1. Keratoacanthoma appears to be a product of the infundibulum of the hair follicle, and various infections have been implicated in its occurrence, including viral sources. Also, mineral oil and tar products historically have had some importance, although the most common denominator appears to be sun exposure. *Id.*

Keratosis follicularis, also known as Darier disease (DD) or Darier-White disease, is a dominantly inherited genodermatosis that is characterized by hyperkeratotic papules in seborrheic regions and various nail abnormalities. Burge SM, J. Am. Acad. Dermatol., 1992, 27(1): 40-50. Abnormal cell-cell adhesion and aberrant epidermal keratinization are its primary features. The majority of DD patients have a clear family history of the disease. The pattern of inheritance is consistent with an autosomal dominant condition. The first skin lesions typically occur in teenage years and are frequently associated with pruritus. Heat, humidity, stress, sunlight and UVB rays have been shown to exacerbate this condition. Even though the severity of DD fluctuates over time, DD is a chronic unremitting condition.

Another type of keratosis is palmoplantar keratoderma (PPK, also known as keratosis palmaris et plantaris), which constitutes a heterogeneous group of disorders characterized by thickening of palms and soles of affected individuals. Lucker GP, et al., Br. J. Dermatol., 1994,131(1):1-14. The condition may be subdivided into hereditary forms, acquired forms, and syndromes with PPK as an associated feature., Hereditary forms may be localized to the hands and feet, or they may be associated with a more generalized skin disorder. Ratnavel RC, et al., Br. J. Dermatol. 1997, 137(4): 485-90. A diffuse pattern of PPK describes uniform involvement of the palmoplantar surface, while focal keratosis refers to localized areas of hyperkeratosis located mainly on pressure points, and punctuate keratoderma features small hyperkeratotic papules, spicules, or nodules on the palms and soles. Acquired forms of PPK are divided into reactive/inflammatory, infective, drug-related, manifestations of systemic disease, keratoderma climacterium, and PPK associated with internal malignancy.

Keratosis pilaris is a common and benign disorder of keratinized hair follicles. Merck Index, 1999 (17th ed.), 833. The disease is characterized by grouped, horny, keratotic follicular papules located predominantly on the lateral aspects of the buttocks, upper arms and thighs. It is usually asymptomatic except for its cosmetic appearance. *Id* Some studies estimate that keratosis pilaris affects 50-80% of all adolescents. The disorder has a familial relationship, which is consistent with autosomal dominant transmission. *Id*

Another type of keratosis is stucco keratosis, which is characterized by keratotic papules that are usually found on distal lower acral extremities of males. Willoughby C. et al., Arch. Dermatol. 1972, 105(6): 859-61. The lesions are usually found in elderly patients. The lesion is asymptomatic and the name stucco keratosis is derived from the "stuck on" appearance of the lesions. Stucco keratosis appears to be produced by thickening of the epidermis, which is typically hyperplastic and usually exophytic with no dysplasia.

### 2.1.2 SKIN CONDITIONS CHARACTERIZED BY OVERGROWTH OF THE EPIDERMIS

### 2.1.2.1. Infections associated with Papilloma Virus

Infections due to papilloma viruses are common skin conditions characterized by overgrowths of the epidermis. Wyngaarden, James B., et al., Cecil Textbook of Medicine, 18 ed. vol. 2, Part XXIV Skin Diseases, p. 2300-2340. Manifestations include, but are limited to, common warts (verrucae vulgaris), palmo-plantar warts, flat warts (verrucae plana), oral warts, focal epithelia hyperplasia, epidermodysplasia verruciformis (EDV), genital warts (condyloma acuminata), Bowen papulosis, Bowen disease, papillomas of the mucosal surfaces, and intraepithelial neoplasias. The viruses infect the basal keratinocyte of the epidermis, presumably through disruptions of the skin or mucosal surface. At this location, the virus remains latent in the cell as a circular episome. As the epidermal cells differentiate and migrate to the surface, the virus is triggered to undergo replication and maturation. The process of virus replication alters the character of the epidermis, resulting in cutaneous or mucosal excrescences known as warts.

Human papilloma viruses (HPVs) are grouped broadly into cutaneous and mucosal types, based on the clinical location of the resulting lesion. Although some overlap exists, most papilloma viruses have distinct anatomic predilections, infecting only certain epidermal sites, such as skin or genital mucosa. A number of genotypes of virus have the potential to transform cells and are associated with epidermal malignancies. The mechanism for transformation is not known, but viral DNA apparently integrates into the genome of the host cell. Histology of condyloma acuminata generally demonstrates disruption of the epidermis with hyperkeratosis, coarse keratohyaline granules, and koilocytes in a prominent granular layer. The epidermis or mucosa of flat condylomata demonstrates acanthosis. The characteristic cytological feature of HPV infection are koilocytes, which are keratinocytes with pyknotic, deeply blue nuclei surrounded by a halo and clear cytoplasm with a paucity of keratohyaline granules. Histology of Bowenoid papulosis reveals psoriasiform hyperplasia and hyperkeratosis of the epidermis. Increased numbers of mitotic figures are observed at all epidermal levels. Keratinocytes display enlarged pleomorphic and hyperchromic nuclei. Histology of common cutaneous warts demonstrates marked hyperkeratosis, acanthosis, perikeratosis, and papillomatosis. Three features used to distinguish warts from other papillomas include the presence of koilocytes, vertical columns of parakeratosis, and foci of clumped keratohyaline granules.

### 2.1.2.2. Arsenical Keratosis

Arsenical keratosis shows thick, compact hyperkeratosis and parakeratosis similar to hypertrophic actinic keratoses. Wyngaarden, James B., et al., Cecil Textbook of Medicine, 18 ed. vol. 2, Part XXIV Skin Diseases, p. 2300-2340. Some epidermal keratinocytes may show atypia histologically. The presence of numerous vacuolated keratinocytes and absence of solar elastosis is are suggestive, although not determinative, of arsenical keratoses.

### 2.1.2.3. Sign of Leser-Trelat

The sign of Leser-Trélat may be more precisely defined as the abrupt appearance of multiple seborrheic keratoses caused by an associated cancer and the rapid increase in their size and number. Wyngaarden, James B., et al., Cecil Textbook of Medicine, 18 ed. vol. 2, Part XXIV Skin Diseases, p. 2300-2340. This definition is parallel to that of malignant acanthosis nigricans; both conditions are obligate paraneoplastic syndromes. Paradoxically, both multiple seborrheic keratoses and acanthosis nigricans are common and rarely linked with cancer. The sign of Leser-Trélat should be considered part of a spectrum of eruptions with internal malignancy that occur relatively commonly with acanthosis nigricans. The associated malignancy is usually aggressive and usually has a poor prognosis. However, neither malignant acanthosis nigricans nor malignancy-induced multiple seborrheic keratoses are cancerous. This syndrome involves epidermal hyperkeratosis, papillomatosis, and acanthosis with cystic inclusion of keratinous material (*e.g.,* horn pseudocysts).

### 2.1.2.4. Warty Dyskeratoma

Warty dyskeratoma (WD) is a solitary, benign, keratotic, epidermal proliferation that commonly presents as an umbilicated lesion usually limited to the head, the neck, or the face. Lesions are sometimes associated with a follicular unit. Wyngaarden, James B., et al., Cecil Textbook of Medicine, 18 ed. vol. 2, Part XXIV Skin Diseases, p. 2300-2340. Mucosal involvement (of oral and genital surfaces) and multiple lesions have been reported.

### 2.1.2.5. Trichostasis Spinulosa

In trichostasis spinulosa (TS), clusters of vellus hairs become embedded within hair follicles, with resultant elevated, dark, spiny papules on the face or trunk. Wyngaarden, James B., et al.,Cecil Textbook of Medicine, 18 ed. vol. 2, Part XXIV Skin Diseases, p. 2300-2340. TS frequently is discovered as an incidental finding, and often it is confused with keratosis pilaris or acne comedones.

Treatment with potassium hydroxide dissolves the keratinous plug, leaving numerous vellus hairs in a characteristic tuft. If a biopsy specimen is obtained, microscopic examination reveals a dilated hair follicle with hyperkeratosis and acanthosis of the follicular epithelium. Inflammatory changes are not a characteristic of TS.

### 2.1.2.6. Erythrokeratodermia Variabilis

Erythrokeratodermia Variabilis (EKV) is a disorder of cornification associated with noninflammatory erythema. Wyngaarden, James B., et al., Cecil Textbook of Medicine, 18 ed. vol. 2, Part XXIV Skin Diseases, p. 2300-2340. Marked hyperkeratosis is present, probably because of an increased proliferation and disturbed differentiation of keratinocytes.

### 2.1.2.7. Ichthyosis Fetalis

Harlequin ichthyosis, the most severe form of congenital ichthyosis, is characterized by a profound thickening of the keratin layer in fetal skin. Wyngaarden, James B., et al., Cecil Textbook of Medicine, 18 ed. vol. 2, Part XXIV Skin Diseases, p. 2300-2340. The affected neonate is born with a massive horny shell of dense platelike scale and contraction abnormalities of the eyes, ears, mouth, and appendages. This armor limits movement and compromises the protective skin barrier, leaving the newborn susceptible to metabolic abnormalities and infection. The term "harlequin" derives from the newborn's facial expression and the triangular and diamond-shaped pattern of hyperkeratosis. The newborn's mouth is pulled wide open, mimicking a clown's smile. The underlying biochemical and genetic abnormality is not understood.

Immunohistocytochemical examination of the skin has revealed characteristic abnormalities in lamellar granule structure and epidermal keratin expression. Stratum corneum is thick and compact. Hyperkeratosis may be more marked around hair follicles compared to the interfollicular epidermis. Parakeratosis and orthokeratosis may be present, particularly on the fingers and toes. Cells within the stratum corneum are abnormally keratinized. Granular, spinous, and basal cell layers appear unremarkable. Inflammatory cells may infiltrate the papillary dermis.

Electron microscopy typically reveals absent or abnormal lamellar granules within the granular layer keratinocytes. Lamellae are absent in the intercellular spaces between the granular cell layer and cornified cell layer. Densely packed lipid droplets and vacuoles are seen within the cytoplasm of the aberrantly cornified cells of the stratum corneum. These lipid inclusions involve entire skin surface, but are more evident on palms and soles. Keratohyalin granules may be absent, normal, or abnormally small and globular. Keratin intermediate filaments within granular cells may have reduced density.

### 2.1.2.8. Knuckle Pads

The histology of knuckle pads shows changes in both the epidermis and dermis. Epidermal abnormalities include hyperkeratosis and mild acanthosis. Wyngaarden, James B., et al., Cecil Textbook of Medicine, 18 ed. vol. 2, Part XXIV Skin Diseases, p. 2300-2340. Dermal changes include slight proliferation of fibroblasts and capillaries in the papillary dermis. Thickened, irregular collagen bundles are present, but there is little accompanying inflammation. These changes resemble those seen in palmar fibromatosis.

### 2.1.2.9. Cutaneous Melanoacanthoma

Microscopic examination of cutaneous melanoacanthoma reveals a proliferation of keratinocytes and melanocytes localized to the epidermis. Wyngaarden, James B., et al., Cecil Textbook of Medicine, 18 ed. vol. 2, Part XXIV Skin Diseases, p. 2300-2340. Acanthosis, hyperkeratosis, papillomatosis, and small horn pearls may be seen. Large dendritic melanocytes with abundant melanin granules are spread throughout the epidermis. In addition, small basaloid and/or spinous keratinocytes are evident in the malpighian layer.

### 2.1.2.10. Porokeratosis

Clonal hyperproliferation of atypical keratinocytes leads to the formation of the comoid lamella, which expands peripherally and forms the raised boundary between abnormal and normal keratinocytes. Wyngaarden, James B., et al., Cecil Textbook of Medicine, 18 ed. vol. 2, Part XXIV Skin Diseases, p. 2300-2340. Local or systemic immunosuppression may allow the development of atypical clones of keratinocytes in individuals who are genetically predisposed. Loss of heterozygosity has been proposed as a mechanism for linear porokeratosis associated with a personal or family history of disseminated superficial actinic porokeratosis (DSAP).

### 2.1.2.11. Squamous Cell Carcinoma

Squamous cell carcinoma may arise *de novo* or from premalignant lesions such as actinic keratosis or intraepidermal carcinoma (carcinoma in situ). Wyngaarden, James B., et al., Cecil Textbook of Medicine, 18 ed. vol. 2, Part XXIV Skin Diseases, p. 2300-2340. Intraepidermal squamous cell carcinoma is characterized by full-thickness epidermal atypia (cellular atypia, loss of polarity, pleomorphic and/or hyperchromatic nuclei, mitotic figures, and parakeratosis).

### 2.1.2.12. Confluent and Reticulated Papillomatosis

In confluent and reticulated papillomatosis (CRP), the epidermis shows compact hyperkeratosis, often associated with the presence of *Pityrosporum* yeast. Wyngaarden, James B., et al., Cecil Textbook of Medicine, 18 ed. vol. 2, Part XXIV Skin Diseases, p. 2300-2340. Other features are less consistent, and include a decreased or absent granular cell layer, papillomatosis, and a stratum spinosum that varies from acanthotic to atrophic. The dermis may contain a perivascular lymphocytic infiltrate. On electron microscopy, some lesions demonstrate alteration in the arrangement and structure of cornified cells, an increased transitional cell layer, an increase in the number of lamellar bodies in the stratum granulosum, and an increased number of melanosomes in the stratum corneum.

### 2.1.2.13. Acrochordon

Acrochordons are characterized by acanthotic, flattened or frondlike epithelium. A hyperplastic epidermis shows papillomatosis, hyperkeratosis, and acanthosis overlying loosely arranged collagen fibers and many capillaries. Wyngaarden, James B., et al., Cecil Textbook of Medicine, 18 ed. vol. 2, Part XXIV Skin Diseases, p. 2300-2340. A papillarylike dermis is composed of loosely arranged collagen fibers and dilated capillaries and lymphatic vessels. Appendages generally are absent. It was thought that acrochordons are marked by decreased numbers of elastic fibers, though one study of elastic tissue in fibroepithelial polyps showed no deficiency of this tissue. Fibroepithelial polyps of the oral mucosa often have slightly flattened epithelium and a tightly packed collagen stroma, which lacks an abundance of fibroblasts.

### 2.1.2.14. Cutaneous Horn

The horn is composed of compacted hyperkeratosis and parakeratosis. Wyngaarden, James B., et al., Cecil Textbook of Medicine, 18 ed. vol. 2, Part XXIV Skin Diseases, p. 2300-2340. The lesion at the base will display features characteristic of the pathologic process responsible for the development of the horn.

### 2.1.2.15. Cowden Disease (Multiple Hamartoma Syndrome)

Mucocutaneous features of Cowden disease (CD) include trichilemmomas, oral mucosal papillomatosus, acral keratoses, and palmoplantar keratoses. Wyngaarden, James B., et al., Cecil Textbook of Medicine, 18 ed. vol. 2, Part XXIV Skin Diseases, p. 2300-2340. CD is associated with the development of several types of malignancy, which is why recognition of individuals with the syndrome is important. In particular, a marked increase is seen in the incidence of breast carcinoma in women and of thyroid carcinoma in both men and women. Reports also exist of several other types of malignancies occurring in patients with CD.

### 2.1.2.16. Dermatosis Papulosa Nigra

Dermatosis papulosa nigra (DPN) is a benign cutaneous condition that is common among blacks. Wyngaarden, James B., et al., Cecil Textbook of Medicine, 18 ed. vol. 2, Part XXIV Skin Diseases, p. 2300-2340. It usually is characterized by multiple, small, hyperpigmented, asymptomatic papules on the face of adult blacks. Histologically, DPN resembles seborrheic keratoses. The condition may be cosmetically undesirable to some patients.

Lesions of DPN have the histologic appearance of seborrheic keratoses; they display hyperkeratosis, irregular acanthosis, keratin-filled invaginations of the epidermis (horn cysts), and marked hyperpigmentation of the basal layer. Although most lesions are of the acanthotic type and show thick interwoven tracts of epidermal cells, they may have a reticulated pattern in which the tracts consist of a double row of basaloid cells.

### 2.1.2.17. Epidermal Nevus Syndrome

Epidermal Nevus Syndrome (ENS) has been classified into four types by clinical, histopathologic, and genetic criteria: linear sebaceous nevus (LSN), linear nevus comedonicus (NC), linear epidermal nevus (LEN), and inflammatory linear verrucous epidermal nevus (ILVEN). Wyngaarden, James B., et al., Cecil Textbook of Medicine, 18 ed. vol. 2, Part XXIV Skin Diseases, p. 2300-2340. Each type may be regarded as part of a syndrome with systemic associations.

Typically, the histologic examination of LEN reveals marked hyperkeratosis, papillomatosis, and acanthosis with rete ridge elongation in a psoriasiform pattern. Changes of epidermolytic hyperkeratosis, acantholytic dyskeratosis, and those resembling verruca vulgaris and comedo formation may also be observed.

Histologic examination of ILVEN reveals a similar psoriasiform hyperplasia of the epidermis, alternating parakeratosis without a granular layer, and orthokeratosis with a thickened granular layer. Occasionally, changes of epidermolytic hyperkeratosis, acantholytic dyskeratosis, and those resembling verruca vulgaris and comedo formation may be noted.

In NC, rudimentary hair follicles are dilated to form epidermal invaginations, which are filled with keratin in concentric lamellae. The follicular walls are composed of several layers of keratinocytes, which occasionally show changes of epidermolytic hyperkeratosis. Scattered hair shafts and small sebaceous lobes may be evident in early specimens; in older specimens, the shafts and lobes, as well as arrector pili muscles, are absent. The interfollicular epidermis is often papillomatous and hyperkeratotic, and ossification may be observed in the dermis.

LSN combines epidermal, follicular, sebaceous, and apocrine gland abnormalities. It reflects the normal sebaceous elements seen in infancy, childhood, and adolescence. Thus, in early life, the lesions are well developed because of maternal hormonal expression, whereas, in childhood, they are underdeveloped and reduced in size and number. At this stage, incomplete and undifferentiated hair structures may be a key to diagnosis, with prominent keratin-filled infundibula and malformed hair germs.

### 2.1.2.18. Ichthyosis Vulgaris

The histological appearances of hereditary ichthyosis and acquired ichthyosis are practically identical. Wyngaarden, James B., et al., Cecil Textbook of Medicine, 18 ed. vol. 2, Part XXIV Skin Diseases, p. 2300-2340. The stratum corneum shows compact hyperkeratosis, though some areas can be laminated. Patchy parakeratosis and occasional follicular plugging is seen. The granular layer usually is one layer thick or absent. The stratum malpighii usually is intact but has a decreased rete-papillae pattern. The eccrine and sebaceous glands, when present, are small, atrophic, and reduced in number. Sweat glands and hair follicles are evident. Most cases show a slight, perivascular, lymphohistiocytic infiltration in the upper dermis. Ichthyosiform sarcoid, a manifestation of acquired ichthyosis in sarcoid patients, has the additional presence of multiple noncaseating granulomas in the dermis.

Ultrastructural studies show reduced or absent keratohyalin granules housed in the granular layer. They appear spongy or crumbly, most likely due to defective keratohyalin synthesis. The hyperkeratotic portions of the stratum corneum have a normal keratin pattern.

### 2.1.2.19. Molluscum Contagiosum

Lesions in Molluscum Contagiosum have a characteristic histopathology. Wyngaarden, James B., et al., Cecil Textbook of Medicine, 18 ed. vol. 2, Part XXIV Skin Diseases, p. 2300-2340. In the fully developed lesion, a crater appears near the surface and gradually extends into lobules that contain hyalinized molluscum bodies (*i.e.,* Henderson-Paterson bodies), which can measure about 35 mm in diameter. Molluscum bodies are membrane-bound sacks that contain numerous MC virions. Downward proliferation of the rete ridges with envelopment by the connective tissue forms the crater. Enlarged deep-purple keratinocytes develop above the basal cell layer of the epidermis. These enlarged keratinocytes contain viral particles, which increase in size as the cells progress upwards. Eventually, the bulk of the viral particles compress the nucleus to the side of the cell to form crescent-shaped nuclei. Intact lesions show little or no inflammatory changes.

Lesions with intradermal rupture of molluscum bodies show an intense dermal inflammatory infiltrate consisting of lymphocytes, histiocytes, and occasional multinucleated foreign body giant cells.

### 2.1.2.20. Prurigo Nodularis

Histology of prurigo nodularis shows a hyperkeratotic epidermis with acanthosis and parakeratosis. Wyngaarden, James B., et al., Cecil Textbook of Medicine, 18 ed. vol. 2, Part XXIV Skin Diseases, p. 2300-2340. Rete ridges are elongated and irregular with a dense dermal infiltrate consisting of neutrophils, eosinophils, histiocytes, and monocytes. Also notable in the dermis are thickened nerve fibers, and fibrosis with thickened collagen bundles.

Thickened nerve fibers are dilated on electron microscopy. Schwann cells show vacuolization and degeneration with no detectable mitochondria. Axons and Schwann cells both show hyperproliferation.

### 2.1.2.21. Acanthosis Nigricans

Acanthosis Nigricans (AN) most likely is caused by factors that stimulate epidermal keratinocyte and dermal fibroblast proliferation. Wyngaarden, James B., et al., Cecil Textbook of Medicine, 18 ed. vol. 2, Part XXIV Skin Diseases, p. 2300-2340. In the benign form of AN, the factor is probably insulin or an insulin-like growth factor that incites the epidermal cell propagation. In malignant AN, the stimulating factor is hypothesized to be a substance secreted either by the tumor or in response to the tumor. Transforming growth factor is structurally similar to epidermal growth factor and is a likely candidate. Exogenous medications also have been implicated as etiologic factors.

### 2.1.3 Scleroderma

Scleroderma is a systemic disease that affects many organ systems. It is most obvious in the skin. However, the gastrointestinal tract, the respiratory, renal, cardiovascular, and genitourinary systems, as well as numerous vascular structures, are involved frequently. The symptoms result from progressive tissue fibrosis and occlusion of the microvasculature by excessive production and deposition of types I and III collagens. As the disease progresses, the skin becomes taut, shiny, and hyper-pigmented; the face becomes mask-like; and telangiectases appear on the fingers, chest, face, lips, and tongue. Subcutaneous calcifications may develop on the fingertips and over bony eminences. The Merck Manual, 17th ed., p. 431.

Proximal scleroderma is characterized by symmetric thickening, tightening, and induration of the skin of the fingers and the skin that is proximal to the metacarpophalangeal or metatarsophalangeal joints. These changes may affect the entire extremity, face, neck, and trunk (thorax and abdomen). Sclerodactyly includes the above major criterion characteristics, but it is limited only to the fingers.

### 2.1.4 Cutaneous Vasculitis

Cutanous vasculitis is an inflammation of blood vessels of the skin. Vasculitis may follow various etiologic mechanisms, but the histologic abnormalities are limited. The inflammation is often segmental, with scattered foci of intense inflammation. Variable degrees of cellular infiltration and necrosis or scarring within one or more layers of the vessel wall occur at the affected sites. The Merck Manual, 17th ed., p. 437.

Any type and size of vessels may be involved. However, most of the pathophysiology can be ascribed to arterial inflammation, with the potential for partial or total vascular occlusion and subsequent tissue necrosis. The intimal and periad-ventitial fibrous response to a focus of intense vessel wall inflammation frequently extends up and down the vessel from the primary result. Intimal hypertrophy and fibrosis or perivasculitis on histology suggest the presence of an adjacent area of vasculitis.

### 2.1.5 ACNE

Acne is a red, irritating skin rash primarily affecting teenagers and young adults. It can, however, occur at all ages. American Academy of Dermatology & Roche Laboratories: *AcneNet.* 2000 Typical acne appears in the oil-producing areas of the body such as the face, chest, and back. Acne can also occur on the neck and upper arms. Several factors contribute to the development of acne. The primary problem is that the abnormal flaking of cells inside the hair follicle leads to the formation of a plug. The plug can enlarge and even rupture the hair follicle. A ruptured hair follicle spills its contents of oil and debris into the skin where it leads to swelling and causes inflammation and redness. In the context of the invention, acne may comprise one or both of the two most commonly-known forms of acne, acne rosacea and acne vulgaris. In another embodiment of the invention, acne does not comprise acne rosacea.

Acne may also comprise acne conglobata. This may be the most severe form of acne vulgaris and is more common in males. It is characterized by numerous large lesions, which are sometimes interconnected, along with widespread blackheads. It can cause severe, irrevocable damage to the skin, and disfiguring scarring. It may be found on the face, chest, back, buttocks, upper arms, and thighs. The age of onset for acne conglobata is usually between 18 and 30 years, and the condition can stay active for many years. As with all forms of acne, the cause of acne conglobata is presently unknown. Treatment may comprise isotretinoin, and although acne conglobata is sometimes resistant to treatment, it can often be controlled through aggressive treatment over time.

Acne may also comprise acne fulminans. This is an abrupt onset of acne conglobata which normally afflicts young men. Symptoms of severe nodulocystic, often ulcerating acne are apparent. As with acne conglobata, extreme, disfiguring scarring is common. Acne fulminans may be unique in that it also includes a fever and aching of the joints. Acne fulminans does not respond well to antibiotics. Isotretinoin and oral steroids may be prescribed.

Acne may also comprise gram-negative folliculitis. This condition is a bacterial infection characterized by pustules and cysts, possibly occurring as a complication resulting from a long term antibiotic treatment of acne vulgaris. Isotretinoin may be effective in combating gram-negative folliculitis.

Acne may also comprise pyoderma faciale. This type of severe acne affects only females, usually between the ages of 20 to 40 years old, and is characterized by painful large nodules, pustules and sores which may leave scarring. It begins abruptly, and may occur on the skin of a woman who has never had acne before. It may be confined to the face, and may not last longer than one year, but can wreak havoc in a very short time.

Acne can have a short-term, potentially lasting psychological effect. Decreased self-esteem and self-confidence can lead to social withdrawal and even depression. Left untreated, severe acne can lead to disfiguring scarring, which can itself be difficult to treat.

### 2.2. CONVENTIONAL TREATMENT

Known methods of treating skin diseases or disorders vary widely from simple measures, such as saltwater soaks and paring, to topical keratolytics, systemic retinoids, and reconstructive surgery with total excision of the keratotic skin.

Medications are the main treatment for acne. Over-the-counter, nonprescription medications can be effective for milder forms of acne. They are in the form of soaps, washes, and cleansers. Prescription medications are useful when acne becomes moderate to severe, or is not controlled by over-the-counter medications. Prescription drugs such as topical retinoids and antibiotics can be used effectively alone or in combination with other prescription and nonprescription medications for acne.

Medical management of keratoses typically begins with educating the patient to limit sun exposure. As the lesions progress, they can be treated with topical keratolytics such as 5-fluorouracil, salicylic acid, lactic acid and urea. Merck Index, 1999 (17th ed.), 827, 833 and 842; and Habib A. Kurwa et al., J. Am. Acad. Dermatol. 1999, 41(3): 414-418. However, this treatment can be temporarily disfiguring with erythematous ulcerations and crust formation, and inflammatory reactions may occur with occlusive dressings and crusting. The application of common topical keratolytics to mucous membranes can also cause increased inflammation and ulceration, which is exacerbated by exposure to sunlight. Lesions flare and become more visible during periods of immune suppression, such as acute sun exposure or chemotherapy, especially with systemic 5-fluorouracil. Consequently, a main disadvantage of this treatment is poor patient compliance. Id

Oral retinoids have been used for many disorders of keratinization due to their influence on epidermal proliferation and differentiation. Merck Index, 1999 (17th ed.), 827 and 833. Topical retinoids such as tretinoin (0.01% gel and 0.1% cream) are effective, but treatment often is limited by skin irritation. *Id.* Topical corticosteroids such as triamcinolone acetonide may be useful for decreasing inflammation by suppressing the migration of polymorphonuclear leukocytes and reversing capillary permeability.

A treatment with photodynamic therapy (PDT) may be considered in patients with contact sensitivity to 5-fluorouracil. Habib A. Kurwa et al., J. Am. Acad. Dermatol. 1999, 41(3): 415. However, this treatment is also painful.

Keratoses can be treated with surgery. Lynn A. Drake et al., J. Am. Acad. Dermatol. 1995, 32:95-98. Cryotherapy and electrosurgery destroy abnormal tissue in the epidermis. Cryotherapy is the most common form of surgical treatment for these lesions. *Id.* and J. Am. Acad. Dermatol. 1994, 31: 648-53. Unfortunately, the patient may need periodic re-treatment for small recurrences or for new lesions. Curettage and cryoablation are the principal options for seborrheic keratosis. Lynn A. Drake et al., J. Am. Acad. Dermatol. 1995, 32:96. Surgical excision, curettage, and electrodesiccation have been the traditional approaches to keratoacanthoma. Dermabrasion may be indicated for removal of the lesions in patients with extensive thickened keratoses of the scalp. *Id.* and George B. Winton et al., J. Am. Acad. Dermatol. 1986, 14:661-668. Laser surgery may be appropriate for the treatment of actinic keratosis of the lips. Lynn A. Drake et al., J. Am. Acad. Dermatol. 1995, 32:97.

Most known therapeutic options are targeted only at the symptoms of skin diseases or disorders, and merely result in short-term improvement. Moreover, they are also attended by unwanted side effects. Therefore, there remains a need for safe and effective methods of treating and managing diseases or disorders.

### 3. SUMMARY OF THE INVENTION

This invention comprises methods of treating, preventing and managing keratoses, scleroderma, cutaneous vasculitis, skin diseases or disorders characterized by overgrowths of the epidermis, acne or wrinkles which comprise administering to a patient in need thereof a therapeutically or prophylactically effective amount of an immunomodulatory compound, or a pharmaceutically acceptable salt, solvate, hydrate, stereoisomer, clathrate, or prodrug thereof. In one embodiment of the invention, acne does not comprise acne rosacea.

One embodiment of the invention comprises the use of an immunomodulatory compound, or a pharmaceutically acceptable salt, solvate, hydrate, stereoisomer, clathrate, or prodrug thereof, in combination with conventional therapies presently used to treat, prevent or manage keratoses, scleroderma, cutaneous vasculitis, skin diseases or disorders characterized by overgrowths of the epidermis, acne or wrinkles. In one embodiment of the invention, acne does not comprise acne rosacea. Conventional therapies include, but are not limited to, treatment with topical keratolytics, topical or systemic retinoids, antibiotics, anti-inflammatory agent, immunomodulatory agent, immunosuppressive agent, herbal products, collagen and botulinum toxin, photodynamic therapy and surgery.

The invention further comprises pharmaceutical compositions, single unit dosage forms, and kits suitable for use in treating, preventing and/or managing keratoses, skin diseases or disorders characterized by overgrowths of the epidermis, scleroderma, cutaneous vasculitis, acne or wrinkles, which comprise an immunomodulatory compound, or a pharmaceutically acceptable salt, solvate, hydrate, stereoisomer, clathrate, or prodrug thereof, and one or more additional active agents. In one embodiment of the invention, acne does not comprise acne rosacea.

### 4. BRIEF DESCRIPTION OF FIGURE

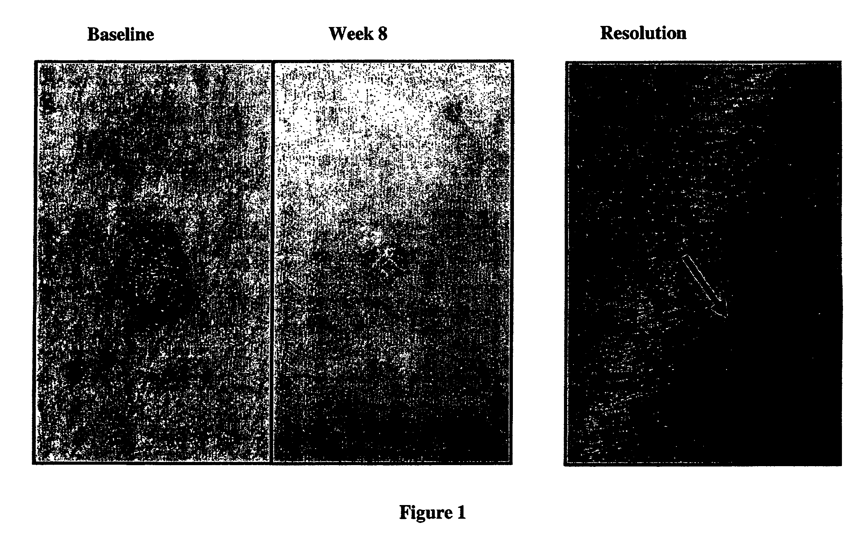

Figure 1 shows a comparison of the responses in patients with actinic keratosis at baseline and after eight weeks of treatment with 3-(4-amino-1-oxo-1,3-dihydroisoindol-2-yl)-piperidine-2,6-dione.

### 5. DETAILED DESCRIPTION OF THE INVENTION

A first embodiment of the invention comprises methods of treating, preventing and managing keratosis which comprise administering to a patient in need of such treatment, prevention or management a therapeutically or prophylactically effective amount of an immunomodulatory compound, or a pharmaceutically acceptable salt, solvate, hydrate, stereoisomer, clathrate, or prodrug thereof.

As used herein, the term "keratosis" refers to any lesion on the epidermis marked by the presence of circumscribed overgrowths of the horny layer, including but not limited to actinic keratosis, seborrheic keratosis, keratoacanthoma, keratosis follicularis (Darier disease), inverted follicular keratosis, palmoplantar keratoderma (PPK, keratosis palmaris et plantaris), keratosis pilaris, and stucco keratosis. The term "actinic keratosis" also refers to senile keratosis, keratosis senilis, verruca senilis, plana senilis, solar keratosis, keratoderma or keratoma. The term "seborrheic keratosis" also refers to seborrheic wart, senile wart, or basal cell papilloma. Keratosis is characterized by one or more of the following symptoms: rough appearing, scaly, erythematous papules, plaques, spicules or nodules on exposed surfaces (*e.g*., face, hands, ears, neck, legs and thorax), excrescences of keratin referred to as cutaneous horns, hyperkeratosis, telangiectasias, elastosis, pigmented lentigines, acanthosis, parakeratosis, dyskeratoses, papillomatosis, hyperpigmentation of the basal cells, cellular atypia, mitotic figures, abnormal cell-cell adhesion, dense inflammatory infiltrates and small prevalence of squamous cell carcinomas.

Another embodiment of the invention comprises methods of treating, preventing and managing skin diseases or disorders characterized by overgrowths of the epidermis, which comprise administering to a patient in need thereof a therapeutically or prophylactically effective amount of an immunomodulatory compound, or a pharmaceutically acceptable salt, solvate, hydrate, stereoisomer, clathrate, or prodrug thereof.

As used herein, the term "skin diseases or disorders characterized by overgrowths of the epidermis" refers to any conditions, diseases or disorders marked by the presence of overgrowths of the epidermis, including but not limited to, infections associated with papilloma virus, arsenical keratoses, sign of Leser- Trélat, warty dyskeratoma (WD), trichostasis spinulosa (TS), erythrokeratodermia variabilis (EKV), ichthyosis fetalis (harlequin ichthyosis), knuckle pads, cutaneous melanoacanthoma, porokeratosis, squamous cell carcinoma, confluent and reticulated papillomatosis (CRP), acrochordons, cutaneous horn, cowden disease (multiple hamartoma syndrome), dermatosis papulosa nigra (DPN), epidermal nevus syndrome (ENS), ichthyosis vulgaris, molluscum contagiosum, prurigo nodularis, and acanthosis nigricans (AN).

Another embodiment of the invention comprises methods of treating, preventing and managing scleroderma which comprise administering to a patient in need of such treatment, prevention or management a therapeutically or prophylactically effective amount of an immunomodulatory compound, or a pharmaceutically acceptable salt, solvate, hydrate, stereoisomer, clathrate, or prodrug thereof.

Another embodiment of the invention comprises methods of treating, preventing and managing cutaneous vasculitis which comprise administering to a patient in need of such treatment, prevention or management a therapeutically or prophylactically effective amount of an immunomodulatory compound, or a pharmaceutically acceptable salt, solvate, hydrate, stereoisomer, clathrate, or prodrug thereof.

Another embodiment of the invention comprises methods of treating, preventing and managing acne, which comprise administering to a patient in need thereof a therapeutically or prophylactically effective amount of an immunomodulatory compound, or a pharmaceutically acceptable salt, solvate, hydrate, stereoisomer, clathrate, or prodrug thereof. In one embodiment of the invention, acne does not comprise acne rosacea.

Still another embodiment of the invention comprises methods of reducing, correcting and managing wrinkles, which comprise administering to a patient in need thereof a therapeutically or prophylactically effective amount of an immunomodulatory compound, or a pharmaceutically acceptable salt, solvate, hydrate, stereoisomer, clathrate, or prodrug thereof.

Further embodiments of the invention comprise methods of treating, preventing, correcting and/or managing keratoses, skin diseases or disorders characterized by overgrowths of the epidermis, scleroderma, cutaneous vasculitis, acne or wrinkles, which comprise administering to a patient in need of such treatment, prevention, correction and/or management a therapeutically or prophylactically effective amount of an immunomodulatory compound, or a pharmaceutically acceptable salt, solvate, hydrate, stereoisomer, clathrate, or prodrug thereof, and a therapeutically or prophylactically effective amount of a second active agent. In one embodiment of the invention, acne does not comprise acne rosacea.

Specific second active agents include keratolytics, retinoids, antibiotics, collagen, botulinum toxin, anti-inflammatory agent, immunomodulatory agents, immunosuppressive agents, herbal products and other chemotherapeutic agents found, for example, in the Physician's Desk Reference, 2003.

Without being limited by theory, it is believed that an immunomodulatory compound can act in complementary or synergistic ways with certain second active agents in the treatment, prevention, correction and/or management of keratoses, skin diseases or disorders characterized by overgrowths of the epidermis, scleroderma, cutaneous vasculitis, acne or wrinkles. In one embodiment of the invention, acne does not comprise acne rosacea. It is further believed that an immunomodulatory compound may reduce or eliminate particular adverse effects associated with conventional therapies, thereby allowing the administration of larger amounts of conventional agents to patients and/or increasing patient compliance.

Another embodiment of the invention comprises methods of reversing, reducing or avoiding adverse effects associated with the administration of chemotherapeutics or therapeutics used to treat keratoses, skin diseases or disorders characterized by overgrowths of the epidermis, scleroderma, cutaneous vasculitis, acne or wrinkles, which comprise administering to the patient in need thereof a therapeutically or prophylactically effective amount of an immunomodulatory compound, a pharmaceutically acceptable salt, solvate, hydrate, stereoisomer, clathrate, or prodrug thereof. In one embodiment of the invention, acne does not comprise acne rosacea.

In still another embodiment, this invention comprises a method of treating and/or managing keratoses, which comprises administering an immunomodulatory compound, a pharmaceutically acceptable salt, solvate, hydrate, stereoisomer, clathrate, or prodrug thereof, in conjunction with (*e.g.* before, during, or after) photodynamic therapy or surgical intervention.

Another embodiment of the invention comprises a pharmaceutical composition for use in treating, preventing, correcting and/or managing keratoses, skin diseases or disorders characterized by overgrowths of the epidermis, scleroderma, cutaneous vasculitis, acne or wrinkles, comprising an immunomodulatory compound, or a pharmaceutically acceptable salt, solvate, hydrate, stereoisomer, clathrate, or prodrug thereof. In one embodiment of the invention, acne does not comprise acne rosacea.

Also comprised by the invention are single unit dosage forms for use in treating, preventing, correcting and/or managing keratoses, skin diseases or disorders characterized by overgrowths of the epidermis, scleroderma, cutaneous vasculitis, acne or wrinkles, comprising an immunomodulatory compound, or a pharmaceutically acceptable salt, solvate, hydrate, stereoisomer, clathrate, or prodrug thereof. In one embodiment of the invention, acne does not comprise acne rosacea.

Another embodiment of the invention comprises a kit for use in treating, preventing, correcting and/or managing keratoses, skin diseases or disorders characterized by overgrowths of the epidermis, scleroderma, cutaneous vasculitis, acne or wrinkles, comprising a pharmaceutical composition comprising an immunomodulatory compound, or a pharmaceutically acceptable salt, solvate, hydrate, stereoisomer, clathrate, or prodrug thereof, and a second active agent. The invention further may comprise kits comprising single unit dosage forms. In one embodiment of the invention, acne does not comprise acne rosacea.

### 5.1. IMMUNOMODULATORY COMPOUNDS

Compounds of the invention can either be commercially purchased or prepared according to the methods described in the patents or patent publications disclosed herein. Further, optically pure compositions can be asymmetrically synthesized or resolved using known resolving agents or chiral columns as well as other standard synthetic organic chemistry techniques. Compounds used in the invention may include immunomodulatory compounds that are racemic, stereomerically enriched or stereomerically pure, and pharmaceutically acceptable salts, solvates, stereoisomers, and prodrugs thereof.

Preferred compounds used in the invention are small organic molecules having a molecular weight less than about 1,000 g/mol, and are not proteins, peptides, oligonucleotides, oligosaccharides or other macromolecules.

As used herein and unless otherwise indicated, the terms "immunomodulatory compounds" and "IMiDs™" (Celgene Corporation) encompasses small organic molecules that markedly inhibit TNF-α, LPS induced monocyte IL1β and IL12, and partially inhibit IL6 production. Specific immunomodulatory compounds are discussed below.

TNF-α is an inflammatory cytokine produced by macrophages and monocytes during acute inflammation. TNF-α is responsible for a diverse range of signaling events within cells. Without being limited by theory, one of the biological effects exerted by the immunomodulatory compounds of the invention is the reduction of synthesis of TNF-α. Immunomodulatory compounds of the invention enhance the degradation of TNF*-*α mRNA.

Further, without being limited by theory, immunomodulatory compounds used in the invention may also be potent co-stimulators of T cells and increase cell proliferation dramatically in a dose dependent manner. Immunomodulatory compounds of the invention may also have a greater co-stimulatory effect on the CD8+ T cell subset than on the CD4+ T cell subset. In addition, the compounds preferably have anti-inflammatory properties, and efficiently co-stimulate T cells. Further, without being limited by a particular theory, immunomodulatory compounds used in the invention may be capable of acting both indirectly through cytokine activation and directly on Natural Killer ("NK") cells, and increase the NK cells' ability to produce beneficial cytokines such as, but not limited to, IFN-γ.

Specific examples of immunomodulatory compounds, include, but are not limited to, cyano and carboxy derivatives of substituted styrenes such as those disclosed in U.S. patent no. 5,929,117; 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. patent nos. 5,874,448 and 5,955,476; the tetra substituted 2-(2,6-dioxopiperdin-3-yl)-1-oxoisoindolines described in U.S. patent no. 5,798,368; 1-oxo and 1,3-dioxo-2-(2,6-dioxopiperidin-3-yl) isoindolines (*e.g*., 4-methyl derivatives of thalidomide), including, but not limited to, those disclosed in U.S. patent nos. 5,635,517, 6,476,052, 6,555,554, and 6,403,613; 1-oxo and 1,3-dioxoisoindolines substituted in the 4- or 5-position of the indoline ring (*e.g.,* 4-(4-amino-1,3-dioxoisoindoline-2-yl)-4-carbamoylbutanoic acid) described in U.S. patent no. 6,380,239; isoindoline-1-one and isoindoline-1,3-dione substituted in the 2-position with 2,6-dioxo-3-hydroxypiperidin-5-yl (*e.g*., 2-(2,6-dioxo-3-hydroxy-5-fluoropiperidin-5-yl)-4-aminoisoindolin-1-one) described in U.S. patent no. 6,458,810; a class of non-polypeptide cyclic amides disclosed in U.S. patent nos. 5,698,579 and 5,877,200; aminothalidomide, as well as analogs, hydrolysis products, metabolites, derivatives and precursors of aminothalidomide, and substituted 2-(2,6-dioxopiperidin-3-yl) phthalimides and substituted 2-(2,6-dioxopiperidin-3-yl)-1-oxoisoindoles such as those described in U.S. patent nos. 6,281,230 and 6,316,471; and isoindole-imide compounds such as those described in U.S. patent application no. 09/972,487 filed on October 5, 2001, U.S. patent application no. 10/032,286 filed on December 21, 2001, and International Application No. PCT/US01/50401 (International Publication No. WO 02/059106). The entireties of each of the patents and patent applications identified herein are incorporated herein by reference. Immunomodulatory compounds do not include thalidomide.

Other specific immunomodulatory compounds of the invention include, but are not limited to, 1-oxo-and 1,3 dioxo-2-(2,6-dioxopiperidin-3-yi) isoindolines substituted with amino in the benzo ring as described in U.S. Patent no. 5,635,517 which is incorporated herein by reference. These compounds have the structure I: 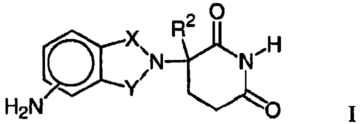 in which one of X and Y is C=O, the other of X and Y is C=O or CH₂, and R² is hydrogen or lower alkyl, in particular methyl. Specific immunomodulatory compounds include, but are not limited to:
1-oxo-2-(2,6-dioxopiperidin-3-yl)-4-aminoisoindoline;
1-oxo-2-(2,6-dioxopiperidin-3-yl)-5-aminoisoindoline;
1-oxo-2-(2,6-dioxopiperidin-3-yl)-6-aminoisoindoline;
1-oxo-2-(2,6-dioxopiperidin-3-yl)-7-aminoisoindoline;
1,3-dioxo-2-(2,6-dioxopiperidin-3-yl)-4-aminoisoindoline; and
1,3-dioxo-2-(2,6-dioxopiperidin-3-yl)-5-aminoisoindoline.

Other specific immunomodulatory compounds of the invention belong to a class of substituted 2-(2,6-dioxopiperidin-3-yl) phthalimides and substituted 2-(2,6-dioxopiperidin-3-yl)-1-oxoisoindoles, such as those described in U.S. patent nos. 6,281,230; 6,316,471; 6,335,349; and 6,476,052, and International Patent Application No. PCT/US97/13375 (International Publication No. WO 98/03502), each of which is incorporated herein by reference. Representative compounds are of formula: 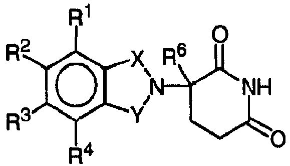 in which:
one of X and Y is C=O and the other of X and Y is C=O or CH₂;
   (i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen or alkyl of 1 to 8 carbon atoms;
R⁶ is hydrogen, alkyl of 1 to 8 carbon atoms, benzyl, or halo;
provided that R⁶ is other than hydrogen if X and Y are C=O and (i) each of R¹, R²,
R³, and R⁴ is fluoro or (ii) one of R¹, R², R³, or R⁴ is amino.

Compounds representative of this class are of the formulas: 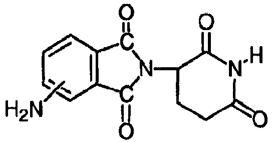 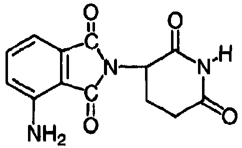 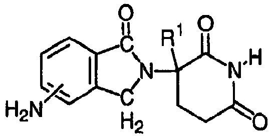 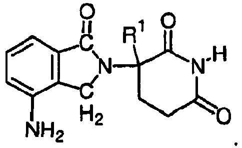 wherein R¹ is hydrogen or methyl. In a separate embodiment, the invention encompasses the use of enantiomerically pure forms (*e.g.* optically pure (R) or (S) enantiomers) of these compounds.

Still other specific immunomodulatory compounds of the invention belong to a class of isoindole-imides disclosed in U.S. Patent Application Publication Nos. US 2003/0096841 and US 2003/0045552, and International Application No. PCT/US01/50401 (International Publication No. WO 02/059106), each of which are incorporated herein by reference. Representative compounds are of formula II: 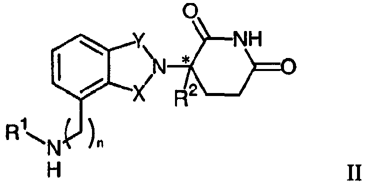 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R¹ is H, (C₁-C₈ )alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)allcenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center.
In specific compounds of formula II, when n is 0 then R¹ is (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(O)OR⁴, (C₁-C₈)alkyl-N(R⁶)₂, (C₁-C₈)alkyl-OR⁵, (C₁-C₈)alkyl-C(O)OR⁵, C(S)NHR³, or (C₁-C₈)alkyl-O(CO)R⁵;
R² is H or (C₁-C₈)alkyl; and
R³ is (C₁-C₈)alkyl, (C₃-C₇)cycloalkyl, (C₂-C₈)alkenyt, (C₂-C₈)alkynyl, benzyl, aryl, (C₀-C₄)alkyt-(C₁ -C₆)heterocycloalkyl, (C₀-C₄)alkyl-(C₂-C₅)heteroaryl, (C₅-C₈)alkyl-N(R⁶)₂ ; (C₀-C₈)alkyl-NH-C(O)O-R⁵; (C₁-C₈)alkyl-OR⁵, (C₁-C₈)alkyl-C(O)OR⁵, (C₁-C₈)alkyl-O(CO)R⁵, or C(O)OR⁵; and the other variables have the same definitions.

In other specific compounds of formula II, R² is H or (C₁-C₄)alkyl.

In other specific compounds of formula II, R¹ is (C₁-C₈)alkyl or benzyl.

In other specific compounds of formula II, R¹ is H, (C₁-C₈)alkyl, benzyl, CH₂OCH₃, CH₂CH₂OCH₃, or

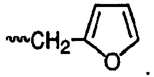

In another embodiment of the compounds of formula II, R¹ is 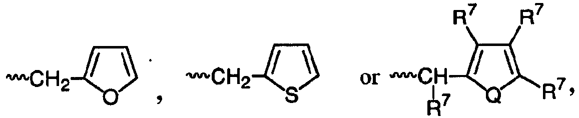 wherein Q is O or S, and each occurrence of R⁷ is independently H,(C₁₋C₈)alkyl, (C₃_ C₇)cycloalkyl, (C₂-C₈)alkenyl, (C₂-C₈)alkynyl, benzyl, aryl, halogen, (C₀₋C₄)alkyl-(C₁-C₆)heterocycloalkyl, (C₀₋C₄)alkyl-(C₂₋C₅)heteroaryl, (C₀₋C₈)alkyl-N(R⁶)₂, (C₁₋C₈)alkyl-OR⁵, (C₁₋C₈)alkyl-C(O)OR⁵, (C₁₋C₈)alkyl-O(CO)R⁵, or C(O)OR⁵, or adjacent occurrences of R⁷ can be taken together to form a bicyclic alkyl or aryl ring.

In other specific compounds of formula II, R¹ is C(O)R³.

In other specific compounds of formula II, R³ is (C₀₋C₄)alkyl-(C₂₋C₅)heteroaryl, (C₁-C₈)alkyl, aryl, or (C₀-C₄)alkyl-OR⁵

In other specific compounds of formula II, heteroaryl is pyridyl, furyl, or thienyl.

In other specific compounds of formula II, R¹ is C(O)OR⁴.

In other specific compounds of formula II, the H of C(O)NHC(O) can be replaced with (C₁-C₄)alkyl, aryl, or benzyl.

Further examples of the compounds in this class include, but are not limited to: [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1*H*-isoindol-4-ylmethyl)-carbamic acid *tert-*butyl ester; 4-(aminomethyl)-2-(2,6-dioxo(3-piperidyl))-isoindoline-1,3-dione; N-(2-(2,6-dioxo-piperidin-3-yl)-1,3-dioxo-2,3-dihydro-1*H*-isoindol-4-ylmethyl)-acetamide; *N*-{(2-(2,6-dioxo(3-piperidyl)-1,3-dioxoisoindolin-4-yl)methyl}cyclopropyl-carboxamide; 2-chloro-*N-*{(2-(2,6-dioxo(3-piperidyl))-1,3-dioxoisoindolin-4-yl)methyl}acetamide; N-(2-(2,6-dioxo(3-piperidyl))-1,3-dioxoisoindolin-4-yl)-3-pyridylcarboxamide; 3- {l-oxo-4-(benzylamino)isoindolin-2-yl}piperidine-2,6-dione; 2-(2,6-dioxo(3-piperidyl))-4-(benzylamino)isoindoline-1,3-dione; *N*-{(2-(2,6-dioxo(3-piperidyl))-1,3-dioxoisoindolin-4-yl)methyl}propanamide; N-{(2-(2,6-dioxo(3-piperidyl))-1,3-dioxoisoindolin-4-yl)methyl}-3-pyridylcarboxamide; N-{(2-(2,6-dioxo(3-piperidyl))-1,3-dioxoisoindolin-4-yl)methyl}heptanamide; *N*- {(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate: *N*-(2-(2,6-dioxo(3-piperidyl))-1.3-dioxoisoindolin-4-yl)pentanamide; *N*-(2(2,6-dioxo(3-piperidyl))-1,3-dioxoisoindolin-4-yl)2-thienylcarboxamide; N-{[2-(2,6-dioxo(3-piperidyl))-1,3-dioxoisoindolin-4-yl] methyl}(butylamino)carboxamide; N-{[2-(2,6-dioxo(3-piperidyl))-1,3-dioxoisoindolin-4-yl] methyl}(octylamino)carboxamide; and N-{[2-(2,6-dioxo(3-piperidyl))-1,3-dioxoisoindolin-4-yl] methyl}(benzylamino)carboxamide.

Still other specific immunomodulatory compounds of the invention belong to a class of isoindole-imides disclosed in U.S. Patent Application Publication Nos. US 2002/0045643, International Publication No. WO 98/54170, and United States Patent No. 6,395,754, each of which is incorporated herein by reference. Representative compounds are of formula III: 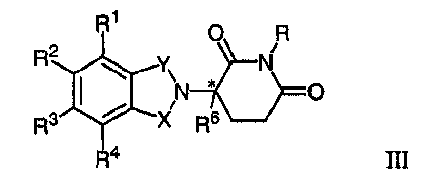 and pharmaceutically acceptable salts, hydrates, solvates, clathrates, enantiomers, diastereomers, racemates, and mixtures of stereoisomers thereof, wherein:
one of X and Y is C=O and the other is CH₂ or C=O;
R is H or CH₂OCOR';
   (i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
R¹⁰ is hydrogen, alkyl of to 8 carbon atoms, or phenyl; and
* represents a chiral-carbon center.

Other representative compounds are of formula: 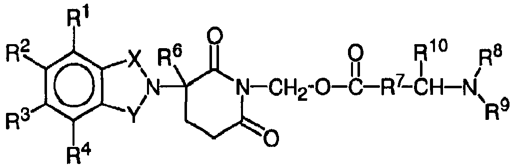 wherein:
one of X and Y is C=O and the other of X and Y is C=O or CH₂;
   (i) each of R¹, R², R³, or R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen or alkyl of 1 to 8 carbon atoms;
R⁶ is hydrogen, alkyl of 1 to 8 carbon atoms, benzo, chloro, or fluoro;
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂ X¹CH₂CH₂- in which X¹ is -O-, -S-, or -NH-;
R¹⁰ is hydrogen, alkyl of to 8 carbon atoms, or phenyl.

Other representative compounds are of formula: 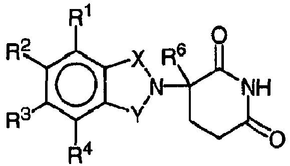 in which
one of X and Y is C=O and the other of X and Y is C=O or CH₂;
each of R¹, R², R³, and R⁴, independently of the others, is halo, alkyl of 1 to 4 carbon atoms, or alkoxy of 1 to 4 carbon atoms or (ii) one of R¹, R², R³, and R⁴ is nitro or protected amino and the remaining of R¹. R², R³, and R⁴ are hydrogen; and
R⁶ is hydrogen, alkyl of 1 to 8 carbon atoms, benzo, chloro, or fluoro.

Other representative compounds are of formula: 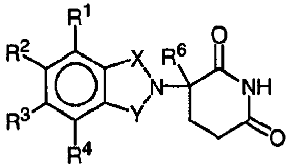 in which:
one of X and Y is C=O and the other of X and Y is C=O or CH₂;
   (i) each of R¹, R², R³, and R⁴, independently of the others, is halo, alkyl of 1 to 4 carbon atoms, or alkoxy of 1 to 4 carbon atoms or (ii) one of R¹, R², R³, and R⁴ is -NHR⁵ and the remaining of R¹, R², R³, and R⁴ are hydrogen;
R⁵ is hydrogen, alkyl of 1 to 8 carbon atoms, or CO-R⁷-CH(R¹⁰)NR⁸R⁹ in which each of R⁷, R⁸, R⁹, and R¹⁰ is as herein defined; and
R⁶ is alkyl of 1 to 8 carbon atoms, benzo, chloro, or fluoro.

Specific examples of the compounds are of formula: 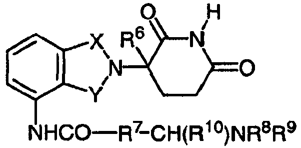 in which:
one of X and Y is C=O and the other of X and Y is C=O or CH₂;
R⁶ is hydrogen, alkyl of 1 to 8 carbon atoms, benzyl, chloro, or fluoro;
R⁷ is m-phenylene, p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X¹CH₂CH₂- in which X¹ is -O-, -S- or -NH-; and
R¹⁰ is hydrogen, alkyl of 1 to 8 carbon atoms, or phenyl.

Preferred immunomodulatory compounds of the invention are 4-(amino)-2-(2,6-dioxo(3-piperidyl))-isoindoline-1,3-dione and 3-(4-amino-1-oxo-1,3-dihydro-isoindol-2-yl)-piperidine-2,6-dione. The compounds can be obtained via standard, synthetic methods (*see e.g*., United States Patent No. 5,635,517, incorporated herein by reference). The compounds are available from Celgene Corporation, Warren, NJ. 4-(Amino)-2-(2,6-dioxo(3-piperidyl))-isoindoline-1,3-dione has the following chemical structure:

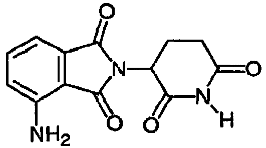

The compound 3-(4-amino-1-oxo-1,3-dihydro-isoindol-2-yl)-piperidine-2,6-dione has the following chemical structure:

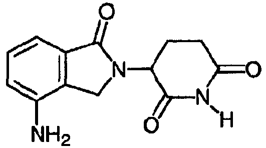

In another embodiment, specific immunomodulatory compounds of the invention encompass polymorphic forms of 3-(4-amino-1-oxo-1,3 dihydro-isoindol-2-yl)-piperidene-2,6-dione such as Form A, B, C, D, E, F, G and H, disclosed in U.S. provisional application no. 60/499,723 filed on September 4, 2003, and U.S. non-provisional application no. 101934,863, filed September 3, 2004, both of which are incorporated herein by reference. For example, Form A of 3-(4-amino-1-oxo-1,3 dihydro-isoindol-2-yl)-piperidene-2,6-dione is an unsolvated, crystalline material that can be obtained from non-aqueous solvent systems. Form A has an X-ray powder diffraction pattern comprising significant peaks at approximately 8, 14.5, 16, 17.5, 20.5, 24 and 26 degrees 2θ, and has a differential scanning calorimetry melting temperature maximum of about 270°C. Form A is weakly or not hygroscopic and appears to be the most thermodynamically stable anhydrous polymorph of 3-(4-amino-1-oxo-1,3 dihydro-isoindol-2-yl)-piperidine-2,6-dione discovered thus far.

Form B of 3-(4-amino-1-oxo-1,3 dihydro-isoindol-2-yl)-piperidene-2,6-dione is a hemihydrated, crystalline material that can be obtained from various solvent systems, including, but not limited to, hexane, toluene, and water. Form B has an X-ray powder diffraction pattern comprising significant peaks at approximately 16, 18, 22 and 27 degrees 2θ, and has endotherms from DSC curve of about 146 and 268°C, which are identified dehydration and melting by hot stage microscopy experiments. Interconversion studies show that Form B converts to Form E in aqueous solvent systems, and converts to other forms in acetone and other anhydrous systems.

Form C of 3-(4-amino-1-oxo-1,3 dihydro-isoindol-2-yl)-piperidene-2,6-dione is a hemisolvated crystalline material that can be obtained from solvents such as, but not limited to, acetone. Form C has an X-ray powder diffraction pattern comprising significant peaks at approximately 15.5 and 25 degrees 2θ, and has a differential scanning calorimetry melting temperature maximum of about 269°C. Form C is not hygroscopic below about 85% RH, but can convert to Form B at higher relative humidities.

Form D of 3-(4-amino-1-oxo-1,3 dihydro-isoindol-2-yl)-piperidene-2,6-dione is a crystalline, solvated polymorph prepared from a mixture of acetonitrile and water. Form D has an X-ray powder diffraction pattern comprising significant peaks at approximately 27 and 28 degrees 2θ, and has a differential scanning calorimetry melting temperature maximum of about 270°C. Form D is either weakly or not hygroscopic, but will typically convert to Form B when stressed at higher relative humidities.

Form E of 3-(4-amino-1-oxo-1,3 dihydro-isoindol-2-yl)-piperidene-2,6-dione is a dihydrated, crystalline material that can be obtained by slurrying 3-(4-amino-1-oxo-1,3 dihydro-isoindol-2-yl)-piperidene-2,6-dione in water and by a slow evaporation of 3-(4-amino-1-oxo-1,3 dihydro-isoindol-2-yl)-piperidene-2,6-dione in a solvent system with a ratio of about 9:1 acetone:water. Form E has an X-ray powder diffraction pattern comprising significant peaks at approximately 20, 24.5 and 29 degrees 2θ, and has a differential scanning calorimetry melting temperature maximum of about 269°C. Form E can convert to Form C in an acetone solvent system and to Form G in a THF solvent system. In aqueous solvent systems, Form E appears to be the most stable form. Desolvation experiments performed on Form E show that upon heating at about 125°C for about five minutes, Form E can convert to Form B. Upon heating at 175°C for about five minutes, Form B can convert to Form F.

Form F of 3-(4-amino-1-oxo-1,3 dihydro-isoindol-2-yl)-piperidene-2,6-dione is an unsolvated, crystalline material that can be obtained from the dehydration of Form E. Form F has an X-ray powder diffraction pattern comprising significant peaks at approximately 19, 19.5 and 25 degrees 29, and has a differential scanning calorimetry melting temperature maximum of about 269°C.

Form G of 3-(4-amino-1-oxo-1,3 dihydro-isoindol-2-yl)-piperidene-2,6-dione is an unsolvated, crystalline material that can be obtained from slurrying forms B and E in a solvent such as, but not limited to, tetrahydrofuran (THF). Form G has an X-ray powder diffraction pattern comprising significant peaks at approximately 21, 23 and 24.5 degrees 2θ, and has a differential scanning calorimetry melting temperature maximum of about 267°C.

Form H of 3-(4-amino-1-oxo-1,3 dihydro-isoindol-2-yl)-piperidene-2,6-dione is a partially hydrated (about 0.25 moles) crystalline material that can be obtained by exposing Form E to 0 % relative humidity. Form H has an X-ray powder diffraction pattern comprising significant peaks at approximately 15, 26 and 31 degrees 2θ, and has a differential scanning calorimetry melting temperature maximum of about 269°C.

Other specific immunomodulatory compounds of the invention include, but are not limited to, 1-oxo-2-(2,6-dioxo-3-fluoropiperidin-3yl) isoindolines and 1,3-dioxo-2-(2,6-dioxo-3-fluoropiperidine-3-yl) isoindolines such as those described in U.S. patent nos. 5,874,448 and 5,955,476, each of which is incorporated herein by reference. Representative compounds are of formula: 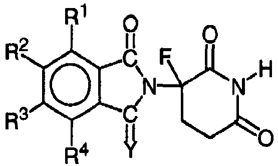 wherein Y is oxygen or H² and
each of R¹, R², R³, and R⁴, independently of the others, is hydrogen, halo, alkyl of 1 to 4 carbon atoms, alkoxy of 1 to 4 carbon atoms, or amino.

Other specific immunomodulatory compounds of the invention include, but are not limited to, the tetra substituted 2-(2,6-dioxopiperdin-3-yl)-1-oxoisoindolines described in U.S. patent no. 5,798,368, which is incorporated herein by reference. Representative compounds are of formula: 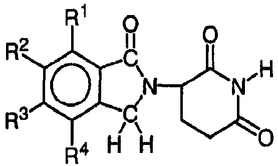 wherein each of R¹, R², R³, and R⁴, independently of the others, is halo, alkyl of 1 to 4 carbon atoms, or alkoxy of 1 to 4 carbon atoms.

Other specific immunomodulatory compounds of the invention include, but are not limited to, 1-oxo and 1,3-dioxo-2-(2,6-dioxopiperidin-3-yl) isoindolines disclosed in U.S. patent no. 6,403,613, which is incorporated herein by reference. Representative compounds are of formula: 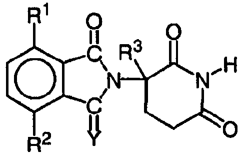 in which
Y is oxygen or H₂,
a first of R¹ and R² is halo, alkyl, alkoxy, alkylamino, dialkylamino, cyano, or carbamoyl, the second of R¹ and R², independently of the first, is hydrogen, halo, alkyl, alkoxy, alkylamino, dialkylamino, cyano, or carbamoyl, and
R³ is hydrogen, alkyl, or benzyl.

Specific examples of the compounds are of formula: 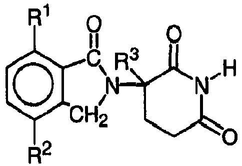 wherein a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl,
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl, and
R³ is hydrogen, alkyl of from 1 to 4 carbon atoms, or benzyl. Specific examples include, but are not limited to, 1-oxo-2-(2,6-dioxopiperidin-3-yl)-4-methylisoindoline.

Other representative compounds are of formula: 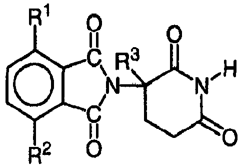 wherein a first of R¹ and R² is halo, alkyl of from 1 to 4 carbon atoms, alkoxy of from 1 to 4 carbon atoms, dialkylamino in which each alkyl is of from 1 to 4 carbon atoms, cyano, or carbamoyl,
the second of R¹ and R², independently of the first, is hydrogen, halo, alkyl of from 1 to 4 carbon atoms, alkoxy of from 1 to 4 carbon atoms, alkylamino in which alkyl is of from 1 to 4 carbon atoms, dialkylamino in which each alkyl is of from 1 to 4 carbon atoms, cyano, or carbamoyl, and
R³ is hydrogen, alkyl of from 1 to 4 carbon atoms, or benzyl.

Specific examples include, but are not limited to, 1-oxo-2-(2,6-dioxopiperidin-3-yl)-4-methylisoindoline.

Other specific immunomodulatory compounds of the invention include, but are not limited to, 1-oxo and 1,3-dioxoisoindolines substituted in the 4- or 5-position of the indoline ring described in U.S. patent no. 6,380,239 and co-pending U.S. application no. 10/900,270, filed July 28, 2004, which are incorporated herein by reference. Representative compounds are of formula: 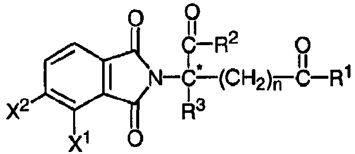 in which the carbon atom designated C* constitutes a center of chirality (when n is not zero and R¹ is not the same as R²); one of X¹ and X² is amino, nitro, alkyl of one to six carbons, or NH-Z, and the other of X¹ or X² is hydrogen; each of R¹ and R² independent of the other, is hydroxy or NH-Z; R³ is hydrogen, alkyl of one to six carbons, halo, or haloalkyl; Z is hydrogen, aryl, alkyl of one to six carbons, formyl, or acyl of one to six carbons; and n has a value of 0, 1, or 2; provided that if X¹ is amino, and n is 1 or 2, then R¹ and R² are not both hydroxy; and the salts thereof.

Further representative compounds are of formula: 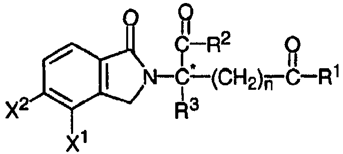 in which the carbon atom designated C* constitutes a center of chirality when n is not zero and R¹ is not R²; one of X¹ and X² is amino, nitro, alkyl of one to six carbons, or NH-Z, and the other of X¹ or X² is hydrogen; each of R¹ and R²independent of the other, is hydroxy or NH-Z; R³ is alkyl of one to six carbons, halo, or hydrogen; Z is hydrogen, aryl or an alkyl or acyl of one to six carbons; and n has a value of 0, 1, or 2.

Specific examples include, but are not limited to, 2-(4-amino-1-oxo-1,3-dihydroisoindol-2-yl)-4-carbamoyl-butyric acid and 4-(4-amino-1-oxo-1,3-dihydro-isoindol-2-yl)-4-cabamoyl-butyric acid, which have the following structures, respectively, and pharmaceutically acceptable salts, solvates, prodrugs, and stereoisomers thereof:

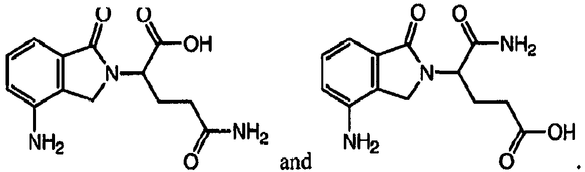

Other representative compounds are of formula: 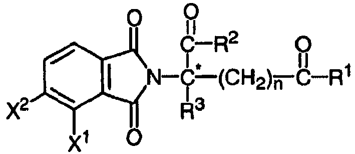 in which the carbon atom designated C* constitutes a center of chirality when n is not zero and R¹ is not R²; one of X¹ and X² is amino, nitro, alkyl of one to six carbons, or NH-Z, and the other of X¹or X² is hydrogen; each of R¹ and R² independent of the other, is hydroxy or NH-Z; R³ is alkyl of one to six carbons, halo, or hydrogen; Z is hydrogen, aryl, or an alkyl or acyl of one to six carbons; and n has a value of 0, 1, or 2; and the salts thereof.

Specific examples include, but are not limited to, 4-carbamoyl-4-{4-[(furan-2-yl-methyl)-amino]-1,3-dioxo-1,3-dihydro-isoindol-2-yl}-butyric acid, 4-carbamoyl-2-{4-[(furan-2-yl-methyl)-aminol-1,3-dioxo-1,3-dihydro-isoindol-2-yl}-butyric acid, 2-{4-[(furan-2-yl-methyl)-amino]-1,3-dioxo-1,3-dihydro-isoindol-2-yl}-4-phenylcarbamoyl-butyric acid, and 2-{4-[(furan-2-yl-methyl)-amino]-1,3-dioxo-1,3-dihydro-isoindol-2-yl}-pentanedioic acid, which have the following structures, respectively, and pharmaceutically acceptablesalts, solvate, prodrugs, and stereoisomers thereof:

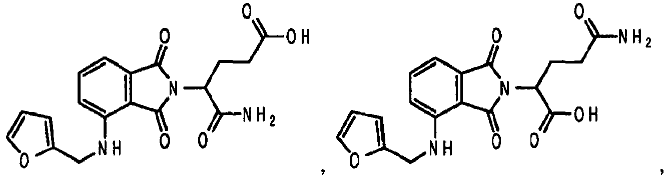

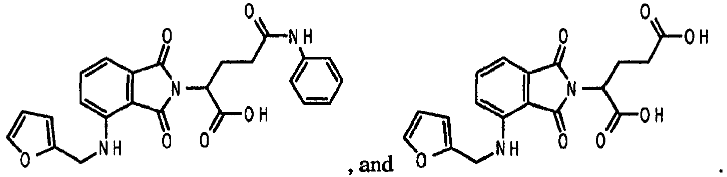

Other specific examples of the compounds are of formula: 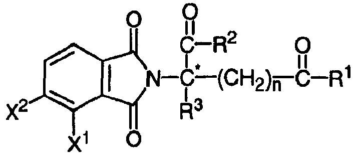
wherein one of X¹ and X² is nitro, or NH-Z, and the other of X¹ or X² is hydrogen;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2;
provided that if one of X¹ and X² is nitro, and n is 1 or 2, then R¹ and R² are other than hydroxy; and
if -COR² and -(CH₂)*ₙ*COR¹ are different, the carbon atom designated C* constitutes a center of chirality. Other representative compounds are of formula: 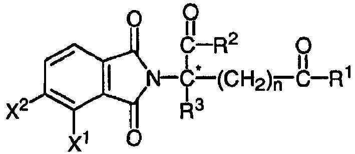
wherein one of X¹ and X² is alkyl of one to six carbons;
each of R¹ and R², independent of the other, is hydroxy or NH-Z;
R³ is alkyl of one to six carbons, halo, or hydrogen;
Z is hydrogen, phenyl, an acyl of one to six carbons, or an alkyl of one to six carbons; and
n has a value of 0, 1, or 2; and
if -COR² and -(CH₂)*ₙ*COR¹ are different, the carbon atom designated C* constitutes a center of chirality.

Still other specific immunomodulatory compounds of the invention include, but are not limited to, isoindoline-1-one and isoindoline-1,3-dione substituted in the 2-position with 2,6-dioxo-3-hydroxypiperidin-5-yl described in U.S. patent no. 6,458,810, which is incorporated herein by reference. Representative compounds are of formula: 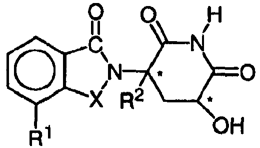 wherein:
the carbon atoms designated constitute centers of chirality;
X is -C(O)- or -CH₂-;
R¹ is alkyl of 1 to 8 carbon atoms or -NHR³;
R² is hydrogen, alkyl of 1 to 8 carbon atoms, or halogen;
and
R³ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms,
benzyl, unsubstituted or substituted with alkyl of 1 to 8 carbon atoms, alkoxy of 1 to 8 carbon atoms, halo, amino, or alkylamino of 1 to 4 carbon atoms, or -COR⁴ in which
R⁴ is hydrogen,
alkyl of 1 to 8 carbon atoms, unsubstituted or substituted with alkoxy of 1 to 8 carbon atoms, halo, amino, or alkylamino of 1 to 4 carbon atoms,
cycloalkyl of 3 to 18 carbon atoms,
phenyl, unsubstituted or substituted with alkyl of 1 to 8 carbon atoms, alkoxy of 1 to 8 carbon atoms, halo, amino, or alkylamino of 1 to 4 carbon atoms, or
benzyl, unsubstituted or substituted with alkyl of 1 to 8 carbon atoms, alkoxy of 1 to 8 carbon atoms, halo, amino, or alkylamino of 1 to 4 carbon atoms.

Compounds of the invention can either be commercially purchased or prepared according to the methods described in the patents or patent publications disclosed herein. Further, optically pure compounds can be asymmetrically synthesized or resolved using known resolving agents or chiral columns as well as other standard synthetic organic chemistry techniques.

As used herein and unless otherwise indicated, the term "pharmaceutically acceptable salt" encompasses non-toxic acid and base addition salts of the compound to which the term refers. Acceptable non-toxic acid addition salts include those derived from organic and inorganic acids or bases know in the art, which include, for example, hydrochloric acid, hydrobromic acid, phosphoric acid, sulfuric acid, methanesulphonic acid, acetic acid, tartaric acid, lactic acid, succinic acid, citric acid, malic acid, maleic acid, sorbic acid, aconitic acid, salicylic acid, phthalic acid, embolic acid, enanthic acid, and the like.

Compounds that are acidic in nature are capable of forming salts with various pharmaceutically acceptable bases. The bases that can be used to prepare pharmaceutically acceptable base addition salts of such acidic compounds are those that form non-toxic base addition salts, *i.e.*, salts containing pharmacologically acceptable cations such as, but not limited to, alkali metal or alkaline earth metal salts and the calcium, magnesium, sodium or potassium salts in particular. Suitable organic bases include, but are not limited to, N,N-dibenzylethylenediamine, chloroprocaine, choline, diethanolamine, ethylenediamine, meglumaine (N-methylglucamine), lysine, and procaine.

As used herein, and unless otherwise specified, the term "solvate" means a compound of the present invention or a salt thereof, that further includes a stoichiometric or non-stoichiometric amount of solvent bound by non-covalent intermolecular forces. Where the solvent is water, the solvate is a hydrate.

As used herein and unless otherwise indicated, the term "prodrug" means a derivative of a compound that can hydrolyze, oxidize, or otherwise react under biological conditions (*in vitro* or *in vivo*) to provide the compound Examples of prodrugs include, but are not limited to, derivatives of immunomodulatory compounds of the invention that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include derivatives of immunomodulatory compounds of the invention that comprise -NO, -NO₂, -ONO, or -ONO₂ moieties. Prodrugs can typically be prepared using well-known methods, such as those described in 1 Burger's Medicinal Chemistry and Drug Discovery, 172-178, 949-982 (Manfred E. Wolff ed., 5th ed. 1995), and Design of Prodrugs (H. Bundgaard ed., Elselvier, New York 1985).

As used herein and unless otherwise indicated, the terms "biohydrolyzable amide," "biohydrolyzable ester," "biohydrolyzable carbamate," "biohydrolyzable carbonate," "biohydrolyzable ureide," "biohydrolyzable phosphate" mean an amide, ester, carbamate, carbonate, ureide, or phosphate, respectively, of a compound that either: 1) does not interfere with the biological activity of the compound but can confer upon that compound advantageous properties *in vivo,* such as uptake, duration of action, or onset of action; or 2) is biologically inactive but is converted *in vivo* to the biologically active compound. Examples of biohydrolyzable esters include, but are not limited to, lower alkyl esters, lower acyloxyalkyl esters (such as acetoxylmethyl, acetoxyethyl, aminocarbonyloxymethyl, pivaloyloxymethyl, and pivaloyloxyethyl esters), lactonyl esters (such as phthalidyl and thiophthalidyl esters), lower alkoxyacyloxyalkyl esters (such as methoxycarbonyl-oxymethyl, ethoxycarbonyloxyethyl and isopropoxycarbonyloxyethyl esters), alkoxyalkyl esters, choline esters, and acylamino alkyl esters (such as acetamidomethyl esters). Examples of biohydrolyzable amides include, but are not limited to, lower alkyl amides, α-amino acid amides, alkoxyacyl amides, and alkylaminoalkylcarbonyl amides. Examples of biohydrolyzable carbamates include, but are not limited to, lower alkylamines, substituted ethylenediamines, amino acids, hydroxyalkylamines, heterocyclic and heteroaromatic amines, and polyether amines.

As used herein, and unless otherwise specified, the term "stereoisomer" encompasses all enantiomerically/stereomerically pure and enantiomerically/stereomerically enriched compounds of this invention.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure when the compound contains 80%, 90%, or 95% or more of one stereoisomer and 20%, 10%, or 5% or less of the counter stereoisomer. In certain cases, a compound of the invention is considered optically active or stereomerically/enantiomerically pure (*i.e.,* substantially the R-form or substantially the S-form) with respect to a chiral center when the compound is about 80% ee (enantiomeric excess) or greater, preferably, equal to or greater than 90% ee with respect to a particular chiral center, and more preferably 95% ee with respect to a particular chiral center.

As used herein, and unless otherwise indicated, the term "stereomerically enriched" or "enantiomerically enriched" encompasses racemic mixtures as well as other mixtures of stereoisomers of compounds of this invention (*e.g.,* R/S = 30/70, 35/65, 40/60, 45/55, 55/45, 60/40, 65/35 and 70/30). Various immunomodulatory compounds of the invention contain one or more chiral centers, and can exist as racemic mixtures of enantiomers or mixtures of diastereomers. This invention encompasses the use of stereomerically pure forms of such compounds, as well as the use of mixtures of those forms. For example, mixtures comprising equal or unequal amounts of the enantiomers of a particular immunomodulatory compounds of the invention may be used in methods and compositions of the invention. These isomers may be asymmetrically synthesized or resolved using standard techniques such as chiral columns or chiral resolving agents. *See, e.g.,* Jacques, J., et al., Enantiomers, Racemates and Resolutions (Wiley-Interscience, New York, 1981); Wilen, S. H., et al., Tetrahedron 33:2725 (1977); Eliel, E. L., Stereochemistry of Carbon Compounds (McGraw-Hill, NY, 1962); and Wilen, S. H., Tables of Resolving Agents and Optical Resolutions p. 268 (E.L. Eliel, Ed., Univ. of Notre Dame Press, Notre Dame, IN, 1972).

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

### 5.2. SECOND ACTIVE AGENTS

One or more second active ingredients can be used in the methods and compositions of the invention together with an immunomodulatory compound, or a pharmaceutically acceptable salt, solvate, hydrate, stereoisomer, clathrate, or prodrug thereof. In a specific embodiment, the second active agents are capable of affecting or inhibiting cell growth or proliferation, removing or improving acne scars, or reducing or correcting wrinkle lines.

Second active agents can be large molecules (*e.g.,* proteins) or small molecules (*e.g.,* synthetic inorganic, organometallic, or organic molecules). Examples of second active agents include, but are not limited to, keratolytics, retinoids, α-hydroxy acids, antibiotics, collagen, botulinum toxin, interferon, immunomodulatory agents, immunosuppressive agents, anti-inflammatory agents, herbal products, and other therapeutics discussed herein. Particular second active agents include, but are not limited to, Xanthium Relieve Surface (for example, any composition comprising one or more of xanthium fruit and magnolia flower), 5-fluorouracil, masoprocol, trichloroacetic acid, salicylic acid, lactic acid, ammonium lactate, urea, isotretinoin, antibiotics, collagen, botulinum toxin, interferon and transretinoic acid.

In one embodiment of the invention, an immunomodulatory compound, or a pharmaceutically acceptable salt, solvate, hydrate, stereoisomer, clathrate, or prodrug thereof is used in combination with a keratolytic. Examples of keratolytics include, but are not limited to, 5-fluorouracil, masoprocol, trichloroacetic acid, salicylic acid, lactic acid, ammonium lactate and urea.

In another embodiment of the invention, an immunomodulatory compound, or a pharmaceutically acceptable salt, solvate, hydrate, stereoisomer, clathrate, or prodrug thereof is used in combination with oral or topical retinoid such as tretinoin and isotretinoin.

In another embodiment of the invention, an immunomodulatory compound, or a pharmaceutically acceptable salt, solvate, hydrate, stereoisomer, clathrate, or prodrug thereof is used in combination with herbal products, including but not limited to, Xanthium Relieve Surface (for example, any combination comprising one or more of xanthium fruit and magnolia flower), Evening primrose oil, Burdock (*Arctium lappa*), Dandelion (*Taraxacum officinale*), Coleus forkolii, Licorice, Yellow dock, Red clover, Sassafras, Sarsaparilla, and Forsythis.

In another embodiment of the invention, an immunomodulatory compound, or a pharmaceutically acceptable salt, solvate, hydrate, stereoisomer, clathrate, or prodrug thereof may be used in combination with an antibiotic, anti-inflammatory agent, immunomodulatory agent, immunosuppressive agents, steroid, and interferon.

In further embodiment of the invention, an immunomodulatory compound, or a pharmaceutically acceptable salt, solvate, hydrate, stereoisomer, clathrate, or prodrug thereof may be used in combination with collagen originated from human, animal, or cadaver skin, including but not limited to, human placental collagen, animal placental collagen, Dermalogen, AlloDerm, Fascia, Cymetra, Autologen, Zyderm, Zyplast, Resoplast, and Isolagen.

In further embodiment of the invention, an immunomodulatory compound, or a pharmaceutically acceptable salt, solvate, hydrate, stereoisomer, clathrate, or prodrug thereof may be used in combination with botulinum toxin, commonly known as Botox.

### 5.3. METHODS OF TREATMENT AND MANAGEMENT

Methods of this invention encompass methods of treating, preventing, correcting and/or managing keratoses, skin diseases or disorders characterized by overgrowths of the epidermis, scleroderma, cutaneous vasculitis, acne or wrinkles. In one embodiment of the invention, acne does not comprise acne rosacea. As used herein, unless otherwise specified, the term "preventing" includes but is not limited to, inhibition or the averting of symptoms associated with the skin diseases, conditions or disorders. Symptoms associated with the skin diseases, conditions or disorders include, but are not limited to, rough appearing, scaly, erythematous papules, plaques, spicules or nodules on exposed surfaces (*e.g.,* face, hands, ears, neck, legs and thorax), excrescences of keratin referred to as cutaneous horns, hyperkeratosis, telangiectasias, elastosis, pigmented lentigines, acanthosis, parakeratosis, dyskeratoses, papillomatosis, hyperpigmentation of the basal cells, cellular atypia, mitotic figures, abnormal cell-cell adhesion, dense inflammatory infiltrates and small prevalence of squamous cell carcinomas. As used herein, unless otherwise specified, the term "treating" refers to the administration of a composition after the onset of symptoms of the skin diseases, conditions or disorders, whereas "preventing" refers to the administration prior to the onset of symptoms, particularly to patients at risk of the skin diseases, conditions or disorders. As used herein and unless otherwise indicated, the term "managing" encompasses preventing the recurrence of the skin diseases, conditions or disorders in a patient who had suffered from the diseases, conditions or disorders, lengthening the time a patient who had suffered from those remains in remission, and/or preventing the occurrence in patients at risk of suffering from those.

Methods encompassed by this invention comprise administering an immunomodulatory compound, or a pharmaceutically acceptable salt, solvate, hydrate, stereoisomer, clathrate, or prodrug thereof to a patient (*e.g.*, a human) suffering, or likely to suffer, from keratoses, skin diseases or disorders characterized by overgrowths of the epidermis, scleroderma, cutaneous vasculitis, acne or wrinkles. In one embodiment of the invention, acne does not comprise acne rosacea. Individuals with family histories of the diseases and those significantly involved in outdoor sports or work are particularly likely to suffer from the diseases.

In one embodiment of the invention, 3-(4-amino-1-oxo-1,3-dihydro-isoindol-2-yl)-piperidine-2,6-dione or 4-(amino)-2-(2,6-dioxo-(3-piperidyl))-isoindoline-1,3-dione is administered orally and in a single or divided daily doses in an amount of from about 0.10 to about 150 mg/day. In one embodiment, 3-(4-amino-1-oxo-1,3-dihydro-isoindol -2-yl)-piperidine-2,6-dione is administered in an amount of from about 5 to about 25 mg per day, or alternatively from about 25 to about 50 mg every other day. In another embodiment, 4-(amino)-2-(2,6-dioxo-(3-piperidyl))-isoindoline-1,3-dione is administered in an amount of from about 0.10 to about 1 mg per day, or alternatively about 5 mg every other day.

In one embodiment of the invention, 3-(4-amino-1-oxo-1,3-dihydro-isoindol-2-yl)-piperidine-2,6-dione is administered orally and in a single or divided daily doses in an amount of from about 0.10 to about 150 mg/day to reduce or correct wrinkles. In a particular embodiment, 3-(4-amino-1-oxo-1,3-dihydro-isoindol-2-yl)-piperidine-2,6-dione is administered in an amount of about 1, 2, 3, 4, S, 6, 7, 8, 9, 10, 1-10, 3-7, or 4-6 mg/day.

### 5.3.1. Combination Therapy With A Second Active Agent

Particular methods of the invention comprise administering 1) an immunomodulatory compound, or a pharmaceutically acceptable salt, solvate, hydrate, stereoisomer, clathrate, or prodrug thereof, and 2) a second active agent or active ingredient. Examples of the second active agents are disclosed herein (*see, e.g.,* section 5.2).

Administration of an immunomodulatory compound, or a pharmaceutically acceptable salt, solvate, hydrate, stereoisomer, clathrate, or prodrug thereof, and a second active agent to a patient can occur simultaneously or sequentially by the same or different routes of administration. The suitability of a particular route of administration employed for a particular active agent will depend on the active agent itself (*e.g.*, whether it can be administered orally without decomposing prior to entering the blood stream) and the disease being treated. A preferred route of administration for an immunomodulatory compound, or a pharmaceutically acceptable salt, solvate, hydrate, stereoisomer, clathrate, or prodrug thereof is oral. Preferred routes of administration for the second active agents or ingredients of the invention are known to those of ordinary skill in the art, for example, in the Physician's Desk Reference, 2003.

In one embodiment, the second active agent is administered topically, intravenously, intralesionally or subcutaneously, and once or twice daily in an amount of from about 1 to about 1000 mg, from about 5 to about 500 mg, from about 10 to about 350 mg, or from about 50 to about 200 mg. The specific amount of the second active agent will depend on the specific agent used, the types of diseases or conditions being treated or managed, the severity and stage of diseases or conditions, and the amounts of an immunomodulatory compound, or a pharmaceutically acceptable salt, solvate, hydrate, stereoisomer, clathrate, or prodrug thereof, and any optional additional active agents concurrently administered to the patient.

In one embodiment, 5-fluorouracil, masoprocol, trichloroacetic acid, salicylic acid, lactic acid, urea, or triamcinolone actonide is administered topically or intralesionally in the form of cream, ointment or solution to affected areas once to three times daily for approximately two to twelve weeks. In one embodiment of the invention, 3-(4-amino-1-oxo-1,3-dihydro-isoindol-2-yl)-piperidine-2,6-dione or 4-(amino)-2-(2,6-dioxo-(3-piperidyl))-isoindvline-1,3-dione may be used in combination with oral retinoids. Oral retinoid such as isotretinoin (Accutane^{®}) may be administered in an amount of 30 to 35 mg/d (0.5 to 1 mg /kg/d) initially, and 25 mg/d (1.8 mg/kg/d) of maintenance dose for 16 weeks followed by a rest period of at least eight weeks. Retinoid can be administered topically as 0.01 % gel and 0.1% cream. In one embodiment, topical corticosteroid such as triamcinolone acetonide 0.1 % cream (Aristocort^{®} or Kenalog^{®}) can be administered until inflammatory component begins to resolve.

In another embodiment, over-the-counter medications such as soaps, washes, and cleansers can be used in combination with an immunomodulatory compound for treating acne. Non-prescription acne medications include, but are not limited to, benzoyl peroxide, salicylic acid (STRI-DEX^{®}), and Lactobacillus acidophilus (DDS-Acidophilus ^{®}).

In a specific embodiment, an immunomodulatory compound is administered in combination with collagen such as human placental collagen, animal placental collagen, Dermalogen, AlloDerm, Fascia, Cymetra, Autologen, Zyderm, Zyplast, Resoplast, and Isolagen. In another specific embodiment, an immunomodulatory compound is administered in combination with Botox, botulinum toxin. These non-surgical, cosmetic methods reduce or correct wrinkles. Small quantities of collagen or Botox are injected into the contracted muscles that cause wrinkles, forcing them to relax, softening the wrinkles or, in some instances, even causing them to disappear.

### 5.3.2. Use With Photodynamic Therapy

This invention encompasses a method of treating and/or managing keratoses, which comprises an immunomodulatory compound, or a pharmaceutically acceptable salt, solvate, hydrate, stereoisomer, clathrate, or prodrug thereof, in conjunction with photodynamic therapy (PDT). PDT uses light to induce cell death. It is believed that when administered in combination with PDT, an immunomodulatory compound, or a pharmaceutically acceptable salt, solvate, hydrate, stereoisomer, clathrate, or prodrug thereof exhibit immunomodulatory activity that can increase the effectiveness of such therapy and reduce patient complications such as pain and redness in affected areas.

This invention encompasses a method of treating, preventing and/or managing keratoses which comprises administering to a patient (*e.g.*, a human) an immunomodulatory compound, or a pharmaceutically acceptable salt, solvate, hydrate, stereoisomer, clathrate, or prodrug thereof, before, during, or after PDT. The PDT may be preceded by administration of topical 5-aminolevulinic acid which accumulates preferentially in the dysplastic cells. The PDT can be undertaken with a PDT 1200 lamp that uses dichroic cut-off filters to emit in the 580 to 740 nm range. Habib A. Kurwa et al., J. Am. Acad. Dermatol. 1999, 41(3): 414-418.

### 5.3.3. Use With Surgical Therapy

This invention encompasses a method of treating and/or managing keratoses, which comprises administering an immunomodulatory compound, or a pharmaceutically acceptable salt, solvate, hydrate, stereoisomer, clathrate, or prodrug thereof, in conjunction with (*e.g.* before, during, or after) surgical treatment. Examples of surgical treatment include, but are not limited to, chemical destruction using topical 5-fluorouracil, masoprocol cream, trichloroacetic acid or other caustic agents, cryosurgery, electrosurgery, curettage, excision, dermabrasion, and laser therapy. Lynn A. Drake et al., J. Am. Acad. Dermatol. 1995, 32:95-8.

### 5.3.4. Cycling Therapy

In certain embodiments, the prophylactic or therapeutic agents of the invention are cyclically administered to a patient. Cycling therapy involves the administration of a first agent for a period of time, followed by the administration of the agent and/or the second agent for a period of time and repeating this sequential administration. Cycling therapy can reduce the development of resistance to one or more of the therapies, avoid or reduce the side effects of one of the therapies, and/or improves the efficacy of the treatment.

In a particular embodiment, prophylactic or therapeutic agents are administered in a cycle of about 16 weeks, about once or twice every day. One cycle can comprise the administration of a therapeutic or prophylactic agent and at least one (1) or three (3) weeks of rest. The number of cycles administered is from about 1 to about 12 cycles, more typically from about two to about ten cycles, and more typically from about two to about eight cycles.

In one embodiment of the invention, 3-(4-amino-1-oxo-1,3-dihydro-isoindol-2-yl)-piperidine-2,6-dione or 4-(amino)-2-(2,6-dioxo-(3-piperidyl))-isoindoline-1,3-dione in an amount of from about 0.1 to about 25 mg/d may be used in combination with oral retinoids in an initial dose of about 30 to about 35 mg/d (0.5 to 1 mg/kg/d) and a maintenance dose of about 25 mg/d (1.8 mg/kg/d) for 16 weeks followed by a rest period of at least eight weeks.

### 5.4. PHARMACEUTICAL COMPOSITIONS AND SINGLE UNIT DOSAGE FORMS

Pharmaceutical compositions can be used in the preparation of individual, single unit dosage forms. Pharmaceutical compositions and dosage forms of the invention comprise an immunomodulatory compound, or a pharmaceutically acceptable salt, solvate, hydrate, stereoisomer, clathrate, or prodrug thereof. Pharmaceutical compositions and dosage forms of the invention can further comprise one or more excipients.

Pharmaceutical compositions and dosage forms of the invention can also comprise one or more additional active ingredients. Examples of optional additional active ingredients are disclosed herein (*see*, *e.g.,* section 5.2).

Single unit dosage forms of the invention are suitable for oral, mucosal (*e.g.*, nasal, sublingual, vaginal, buccal, or rectal), or parenteral (*e.g.*, subcutaneous, intravenous, bolus injection, intramuscular, or intraarterial), transdermal or transcutaneous administration to a patent. Examples of dosage forms include, but are not limited to: tablets; caplets; capsules, such as soft elastic gelatin capsules; cachets; troches; lozenges; dispersions; suppositories; powders; aerosols (*e.g.,* nasal sprays or inhalers); gels; liquid dosage forms suitable for oral or mucosal administration to a patient, including suspensions (*e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g.*, crystalline or amorphous solids) that can be reconstituted to provide liquid dosage forms suitable for parenteral administration to a patient.

The composition, shape, and type of dosage forms of the invention will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms encompassed by this invention will vary from one another will be readily apparent to those skilled in the art. *See, e.g.,* Remington's Pharmaceutical Sciences, 18th ed., Mack Publishing, Easton PA (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, this invention encompasses pharmaceutical compositions and dosage forms that contain little, if any, lactose other mono- or di-saccharides. As used herein, the term "lactose-free" means that the amount of lactose present, if any, is insufficient to substantially increase the degradation rate of an active ingredient.

Lactose-free compositions of the invention can comprise excipients that are well known in the art and are listed, for example, in the U.S. Pharmacopeia (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (*e.g.*, 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e.g.*, vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms of the invention comprise 3-(4-amino-1-oxo-1,3-dihydro-isoindol-2-yl)-piperidine-2,6-dione or 4-(amino)-2-(2,6-dioxo-(3-piperidyl))-isoindoline-1,3-dione, or a pharmaceutically acceptable salt, solvate, hydrate, clathrate, or prodrug thereof in an amount of about 0.1, 1, 2, 5, 7.5, 10, 12.5, 15, 17.5, 20, 25, 50, 100, 150 or 200 mg. In a specific embodiment, a preferred dosage form comprises 3-(4-amino-1-oxo-1,3-dihydro-isoindol-2-yl)- piperidine-2,6-dione in an amount of about 5, 10, 25 or 50 mg. In another specific embodiment, a preferred dosage form comprises 4-(amino)-2-(2,6-dioxo-(3-piperidyl))-isoindoline-1,3-dione in an amount of about 1, 2, 5, 10, 25 or 50 mg. Typical dosage forms comprise the second active ingredient in an amount of 1 to about 1000 mg, from about 5 to about 500 mg, from about 10 to about 350 mg, or from about 50 to about 200 mg. Of course, the specific amount of the second active ingredient will depend on the specific agent used, the type of diseases or conditions being treated or managed, and the amounts of 3-(4-amino-1-oxo-1,3-dihydro-isoindol-2-yl)-piperidine-2,6-dione, 4-(amino)-2-(2,6-dioxo-(3-piperidyl))-isoindoline-1,3-dione and any optional additional active agents concurrently administered to the patient.

### 5.4.1. ORAL DOSAGE FORMS

Pharmaceutical compositions of the invention that are suitable for oral administration can be presented as discrete dosage forms, such as, but are not limited to, tablets (*e.g.*, chewable tablets), caplets, capsules, and liquids (*e.g.*, flavored syrups). Such dosage forms contain predetermined amounts of active ingredients, and may be prepared by methods of pharmacy well known to those skilled in the art. *See generally,* Remington's Pharmaceutical Sciences, 18th ed., Mack Publishing, Easton PA (1990).

Typical oral dosage forms of the invention are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.*, powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.,* ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g.,* Nos. 2208, 2906, 2910), microcrystalline cellulose, and mixtures thereof.

Suitable forms of microcrystalline cellulose include, but are not limited to, the materials sold as AVICEL-PH-101, AVICEL-PH-102, AVICEL-PH-103, AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Examples of fillers suitable for use in the pharmaceutical compositions and dosage forms disclosed herein include, but are not limited to, talc, calcium carbonate (*e.g.*, granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.*, peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about one weight percent of the pharmaceutical compositions or dosage forms into which they are incorporated.

A preferred solid oral dosage form of the invention comprises 3-(4-amino-1-oxo-1,3-dihydro-isoindol-2-yl)-piperidine-2,6-dione or 4-(amino)-2-(2,6-dioxo-(3-piperidyl))-isoindoline-1,3-dione, anhydrous lactose, AVICEL-PH-102, croscarmellose sodium, magnesium stearate and gelatin.

### 5.4.2. DELAYED RELEASE DOSAGE FORMS

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g.*, adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### 5.4.3. PARENTERAL DOSAGE FORMS

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention. For example, cyclodextrin and its derivatives can be used to increase the solubility of 3-(4-amino-1-oxo-1,3-dihydro-isoindol-2-yl)-piperidine-2,6-dione or 4-(amino)-2-(2,6-dioxo-(3-piperidyl))-isoindoline-1,3-dione, and its derivatives. *See, e.g.,* U.S. Patent No. 5,134,127, which is incorporated herein by reference.

### 5.4.4. TOPICAL AND MUCOSAL DOSAGE FORMS

Topical and mucosal dosage forms of the invention include, but are not limited to, sprays, aerosols, solutions, emulsions, suspensions, lotions, creams, ointments or other forms known to one of skill in the art. *See, e.g.,* Remington's Pharmaceutical Sciences, 16th, and 18th eds., Mack Publishing, Easton PA (1980 & 1990); and Introduction to Pharmaceutical Dosage Forms, 4th ed., Lea & Febiger, Philadelphia (1985).

In one embodiment of the invention, dosage forms of an immunomodulatory compound, or a pharmaceutically acceptable salt, solvate, hydrate, stereoisomer, clathrate, or prodrug thereof can be formulated as creams, lotions, or ointments to be topically applied.

Suitable excipients (*e.g.,* carriers and diluents) and other materials that can be used to provide topical and mucosal dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. *See, e.g.,* Remington's Pharmaceutical Sciences, 16th and 18th eds., Mack Publishing, Easton PA (1980 & 1990).

The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### 5.4.5. KITS

Typically, active ingredients of the invention are preferably not administered to a patient at the same time or by the same route of administration. This invention therefore encompasses kits which, when used by the medical practitioner, can simplify the administration of appropriate amounts of active ingredients to a patient.

A typical kit of the invention comprises a dosage form of an immunomodulatory compound, or a pharmaceutically acceptable salt, solvate, hydrate, stereoisomer, clathrate, or prodrug thereof. Kits encompassed by this invention can further comprise second active agents such as antibiotics, collagen, botulinum toxin (Botox), 5-fluorouracil, masoprocol, trichloroacetic acid, salicylic acid, lactic acid, urea, isotretinoin, interferon, or a combination thereof. Examples of the second active agents include, but are not limited to, those disclosed herein (*see, e.g.,* section 5.2).

Kits of the invention can further comprise devices that are used to administer the active ingredients. Examples of such devices include, but are not limited to, syringes, drip bags, patches, and inhalers.

Kits of the invention can further comprise pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### 6. EXAMPLES

The following studies are intended to further illustrate the invention without limiting its scope.

### 6.1. PHARMACOLOGY AND TOXICOLOGY STUDIES

A series of non-clinical pharmacology and toxicology studies have been performed to support the clinical evaluation of an immunomodulatory compound such as 3-(4-amino-1-oxo-1,3-dihydro-isoindol-2-yl)- piperidine-2,6-dione or 4-(amino)-2-(2,6-dioxo-(3-piperidyl))-isoindoline-1,3-dione in human subjects. These studies were performed in accordance with internationally recognized guidelines for study design and in compliance with the requirements of Good Laboratory Practice (GLP), unless otherwise noted.

The pharmacological properties of 3-(4-amino-1-oxo-1,3-dihydro-isoindol-2-yl)-piperidine-2,6-dione or 4-(amino)-2-(2,6-dioxo-(3-piperidyl))-isoindoline-1,3-dione, including activity comparisons with thalidomide, are characterized in *in vitro* studies. Studies examined the effects of 3-(4-amino-1-oxo-1,3-dihydro-isoindol-2-yl)-piperidine-2,6-dione, 4-(amino)-2-(2,6-dioxo-(3-piperidyl))-isoindoline-1,3-dione or thalidomide on the production of various cytokines. 3-(4-Amino-1-oxo-1,3-dihydro-isoindol-2-yl)-piperidine-2,6-dione or 4-(amino)-2-(2,6-dioxo-(3-piperidyl))-isoindoline-1,3-dione markedly inhibited the secretion of the pro-inflammatory cytokines TNF-α, IL1β and IL6. The compound of the invention enhanced the degradation of TNF-α mRNA. The compound increased the secretion of the anti-inflammatory cytokine IL10. The compound induced T-cell proliferation and IL2 and IFN-γ production. In all studies, 3-(4-amino-1-oxo-1,3-dihydro-isoindol-2-yl)-piperidine-2,6-dione or 4-(amino)-2-(2,6-dioxo-(3-piperidyl))-isoindoline-1,3-dione was 50 to 2,000 times more potent than thalidomide.

In addition, a safety pharmacology study of 3-(4-amino-1-oxo-1,3-dihydro-isoindol -2-yl)-piperidine-2,6-dione or 4-(amino)-2-(2,6-dioxo-(3-piperidyl))-isoindoline-1,3-dione was conducted in dogs. The effects of the compound on cardiovascular and respiratory functions were examined in anesthetized dogs. One group of Beagle dogs (2/sex/group) received three doses of vehicle only and the other received three ascending doses of 2, 10 and 20 mg/kg of the compound. No animals died in this study. The cardiovascular and respiratory changes induced by the compound were minimal at all doses when compared to the vehicle control group. A small transient increase in arterial blood pressure was observed following the administration of 2 mg/kg of the compound but it was not seen at higher doses. Deviations in femoral blood flow, respiratory parameters and QTc interval were common to both the control and treated groups and were not considered treatment-related.

### 6.2. MODULATION OF CYTOKINE PRODUCTION

Inhibitions of TNF-α production following LPS-stimulation of human PBMC and human whole blood by an immunomodulatory compound such as 3-(4-amino-1-oxo-1,3-dihydro-isoindol-2-yl)-piperidine-2,6-dione, 4-(amino)-2-(2,6-dioxo-(3-piperidyl))-isoindoline-1,3-dione or thalidomide were investigated *in vitro.* The IC₅₀'s of 4-(amino)-2-(2,6-dioxo-(3-piperidyl))-isoindoline-1,3-dione for inhibiting production of TNF-α following LPS-stimulation of PBMC and human whole blood were -24 nM (6.55 ng/mL) and -25 nM (6.83 ng/mL), respectively. The IC₅₀'s of 3-(4-amino-1-oxo-1,3-dihydroisoindol-2-yl)-piperidine-2,6-dione for inhibiting production of TNF-α following LPS-stimulation of PBMC and human whole blood were ∼100 nM (25.9 ng/mL) and -480 nM (103.6 ng/mL), respectively. Thalidomide, in contrast, had an IC₅₀ of ∼194 µM (50.1 µg/mL) for inhibiting production of TNF-α following LPS-stimulation of PBMC.

*In vitro* studies suggest a pharmacological activity profile for 3-(4-amino-1-oxo-1,3 -dihydro-isoindol-2-yl)-piperidine-2,6-dione or 4-(amino)-2-(2,6-dioxo-(3-piperidyl))-isoindoline-1,3-dione is similar to, but 50 to 2,000 times more potent than, thalidomide. The pharmacological effects of 3-(4-amino-1-oxo-1,3-dihydro-isoindol-2-yl)-piperidine -2,6-dione or 4-(amino)-2-(2,6-dioxo-(3-piperidyl))-isoindoline-1,3-dione derive from its action as an inhibitor of cellular response to receptor-initiated trophic signals (*e.g.,* IGF-1, VEGF, cyclooxygenase-2), and other activities. As a result, 3-(4-amino-1-oxo-1,3 -dihydro-isoindol-2-yl)-piperidine-2,6-dione or 4-(amino)-2-(2,6-dioxo-(3-piperidyl))-isoindoline-1,3-dione suppresses the generation of inflammatory cytokines, down-regulates adhesion molecules and apoptosis inhibitory proteins (*e.g.*, cFLIP, cIAP), promotes sensitivity to death-receptor initiated programmed cell death, and suppresses angiogenic response.

In addition, it has been shown that 3-(4-amino-1-oxo-1,3-dihydro-isoindol-2-yl) -piperidine-2,6-dione or 4-(amino)-2-(2,6-dioxo-(3-piperidyl))-isoindoline-1,3-dione is approximately 50 to 100 times more potent than thalidomide in stimulating the proliferation of T-cells following primary induction by T-cell receptor (TCR) activation. The compounds are also approximately 50 to 100 times more potent than thalidomide in augmenting the production of IL2 and IFN-γ following TCR activation of PBMC (IL2) or T-cells (IFN-γ). Further, the compounds exhibited dose-dependent inhibition of LPS-stimulated production of the pro-inflammatory cytokines TNF-α, IL1β and IL6 by PBMC while they increased production of the anti-inflammatory cytokine IL10.

### 6.3. CLINICAL STUDIES

### 6.3.1 CLINICAL STUDIES IN KERATOSIS PATIENTS

3-(4-Amino-1-oxo-1,3-dihydro-isoindol-2-yl)-piperidine-2,6-dione or 4-(amino)-2-(2,6-dioxo-(3-piperidyl))-isoindoline-1,3-dione is administered in an amount of from about 1 to about 25 mg per day to patients with keratoses for eight weeks, and the responses are subsequently assessed. For example, clinical studies are performed for 3-(4-amino-1-oxo-1,3-dihydro-isoindol-2-yl)-piperidine-2,6-dione in patients with actinic or senile keratoses. Patients receive continuous treatment with the compound at a oral dose of 25 mg daily. Responses are assessed at baseline and after eight weeks of treatments, and the results are shown in Figure 1. The results of this study indicate that the compound has remarkable clinical benefit in patients with actinic or senile keratoses.

### 6.3.2 CLINICAL STUDIES IN KERATOSIS PATIENTS

3-(4-Amino-1-oxo-1,3-dihydro-isoindol-2-yl)-piperidine-2,6-dione or 4-(amino)-2-(2,6-dioxo-(3-piperidyl))-isoindoline-1,3-dione is administered in an amount of from about 1 to about 25 mg per day to reduce or correct wrinkles of patients. For example, clinical studies are performed for 3-(4-amino-1-oxo-1,3-dihydro-isoindol-2-yl)-piperidine-2,6-dione in patients with wrinkles. Patients receive continuous treatment with the compound at a oral dose of about 5 mg daily. The results indicate that the compound has remarkable clinical benefit in patients with wrinkles.

### 6.3.3 CLINICAL STUDIES IN KERATOSIS PATIENTS

3-(4-Amino-1-oxo-1,3-dihydro-isoindol-2-yl)-piperidine-2,6-dione or 4-(amino)-2-(2,6-dioxo-(3-piperidyl))-isoindoline-1,3-dione is administered in an amount of from about 1 to about 25 mg per day to treat acne. Patients receive continuous treatment with the compound at a oral dose of about 5 mg daily. The results show significant improvements in acne such as reduction in size and number of visible cutaneous infections and/or pimples.

Embodiments of the invention described herein are only representative of the invention. The full scope of the invention is better understood with reference to the attached claims.

In the following, certain items of the invention are shown.
1. A method of treating, preventing or managing a skin disease or disorder characterized by overgrowth of the epidermis, keratosis, acne that does not comprise acne rosacea, or a wrinkle, which comprises administering to a patient in need of such treatment, prevention or management a therapeutically or prophylactically effective amount of an immunomodulatory compound, or a pharmaceutically acceptable salt, solvate, or stereoisomer thereof.
2. A method of treating, preventing or managing senile keratosis, which comprises administering to a patient in need of such treatment, prevention or management a therapeutically or prophylactically effective amount of an immunomodulatory compound, or a pharmaceutically acceptable salt, solvate, or stereoisomer thereof.
3. The method of item 1, further comprising administering to the patient a therapeutically or prophylactically effective amount of a second active ingredient.
4. The method of item 3, wherein the second active ingredient is capable of affecting or inhibiting cell growth or proliferation, removing or improving acne scar, or reducing or correcting wrinkle line.
5. The method of item 3, wherein the second active ingredient is a keratolytic, retinoid, anti-inflammatory agent, immunosuppressive agent, herbal product, antibiotic, collagen, botulinum toxin, interferon, or immunomodulatory agent.
6. The method of item 3, wherein the second active ingredient is xanthium fruit, magnolia flower, 5-fluorouracil, masoprocol, trichloroacetic acid, salicylic acid, lactic acid, ammonium lactate, urea, isotretinoin, α-hydroxy acid, triamcinolone acetonide, collagen, botulinum toxin, or interferon.
7. A method of treating or managing keratosis, which comprises administering to a patient in need of such treatment or management a therapeutically or prophylactically effective amount of an immunomodulatory compound, or a pharmaceutically acceptable salt, solvate, or stereoisomer thereof, before, during or after performing photodynamic therapy or surgery in the patient.
8. The method of item 1, wherein the immunomodulatory compound is 4-(amino)-2-(2,6-dioxo(3-piperidyl))-isoindoline-1,3-dione.
9. The method of item 8, wherein the immunomodulatory compound is enantiomerically pure.
10. The method of item 1, wherein the immunomodulatory compound is 3-(4-amino-1-oxo-1,3-dihydro-isoindol-2-yl)-piperidine-2,6-dione.
11. The method of item 10, wherein the immunomodulatory compound is enantiomerically pure.
12. The method of item 1, wherein the immunomodulatory compound is of formula (I): 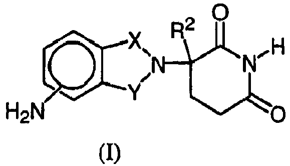 wherein one of X and Y is C=O, the other of X and Y is C=O or CH₂, and R² is hydrogen or lower alkyl.
13. The method of item 12, wherein the immunomodulatory compound is enantiomerically pure.
14. The method of item 1, wherein the immunomodulatory compound is of formula (II): 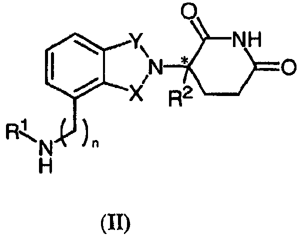 wherein
   one of X and Y is C=O and the other is CH₂ or C=O;
   R¹ is H, (C₁₋C₈)alkyl, (C₃₋C₇)cycloalkyl, (C₂₋ C₈)alkenyl, (C₂₋C₈)alkynyl, benzyl, aryl, (C₀₋C₄)alkyl-(C₁₋C₆)heterocycloalkyl, (C₀₋C₄)alkyl-(C₂-C₅)heteroaryl, C(O)R³, C(S)R³, C(O)OR⁴, (C₁₋C₈)alkyl-N(R⁶)₂, (C₁₋C₈)alkyl-OR⁵, (C₁₋C₈)alkyl-C(O)OR⁵, C(O)NHR³, C(S)NHR³, C(O)NR³R^{3'}, C(S)NR³R^{3'} or (C₁₋C₈)alkyl-O(CO)R⁵;
   R² is H, F, benzyl, (C₁₋C₈)alkyl, (C₂₋C₈)alkenyl, or (C₂₋C₈)alkynyl;
   R³ and R^{3'} are independently (C₁₋C₈)alkyl, (C₃₋C₇)cyclalkyl, (C₂₋C₈)alkenyl, (C₂-C₈)alkynyl, benzyl, aryl, (C₀₋C₄)alkyl-(C₁₋C₆)heterocycloalkyl, (C₀₋C₄)alkyl-(C₂-C₅)heteroaryl, (C₀₋C₈)alkyl-N(R⁶)₂, (C₁₋C₈)alkyl-OR⁵, (C₁₋C₈)alkyl-C(O)OR⁵, (C₁₋C₈)alkyl-O(CO)R⁵, or C(O)OR⁵;
   R⁴ is (C₁₋C₈)alkyl, (C₂₋C₈)alkenyl, (C₂₋C₈)alkynyl, (C₁₋C₄)alkyl-OR⁵, benzyl, aryl, (C₀₋C₄)alkyl-(C₁₋C₆)heterocycloalkyl, or (C₀₋C₄)alkyl-(C₂₋C₅)heteroaryl;
   R⁵ is (C₁₋C₈)alkyl, (C₂₋C₈)alkenyl, (C₂₋C₈)alkynyl, benzyl, aryl, or (C₂₋C₅)heteroaryl;
   each occurrence of R⁶ is independently H, (C₁₋C₈)alkyl, (C₂₋C₈)alkenyl, (C₂-C₈)alkynyl, benzyl, aryl, (C₂₋C₅)heteroaryl, or (C₀₋C₈)alkyl-C(O)O-R⁵ or the R⁶ groups join to form a heterocycloalkyl group;
   n is 0 or 1; and
   * represents a chiral-carbon center.
15. The method of item 14, wherein the immunomodulatory compound is enantiomerically pure.
16. The method of item 1, wherein the immunomodulatory compound is a cyano or carboxyl derivative of a substituted styrene, 1-oxo-2-(2,6-dioxo-3-fluoropiperidin-3yl) isoindoline, 1,3-dioxo-2-(2,6-dioxo-3-fluoropiperidine-3-yl) isoindoline, or tetra substituted 2-(2,6-dioxopiperdin-3-yl)-1-oxoisoindoline.
17. The method of item 16, wherein the immunomodulatory compound is enantiomerically pure.
18. A pharmaceutical composition comprising an immunomodulatory compound, or a pharmaceutically acceptable salt, solvate, or stereoisomer thereof, and a second active ingredient capable of affecting or inhibiting cell growth or proliferation, removing or improving acne scar, or reducing or correcting wrinkle line.
19. The pharmaceutical composition of item 18, wherein the second active ingredient is a keratolytic, retinoid, anti-inflammatory agent, antibiotic, collagen, botulinum toxin, interferon, herbal product, immunosuppressive agent or immunomodulatory agent.
20. The pharmaceutical composition of item 19, wherein the second active ingredient is xanthium fruit, magnolia flower, 5-fluorouracil, masoprocol, trichloroacetic acid, salicylic acid, lactic acid, ammonium lactate, urea, isotretinoin, α-hydroxy acid, triamcinolone acetonide, collagen, botulinum toxin, or interferon.
21. A single unit dosage form comprising the pharmaceutical composition of item 19.
22. A kit comprising:
   a pharmaceutical composition comprising an immunomodulatory compound, or a pharmaceutically acceptable salt, solvate, or stereoisomer thereof; and
   a pharmaceutical composition comprising a keratolytic, retinoid, anti-inflammatory agent, antibiotic, collagen, botulinum toxin, interferon, herbal product, immunosuppressive agent or immunomodulatory agent.
23. The kit of item 22 comprising:
   a pharmaceutical composition comprising 3-(4-amino-1-oxo-1,3-dihydro -isoindol-2-yl)-piperidine-2,6-dione or a pharmaceutically acceptable salt or solvate; and
   placental collagen or botulinum toxin.
24. The method of item 1, wherein said acne does not comprise acne rosacea.

## Claims

1. A compound, which is 3-(4-amino-1-oxo-1,3-dihydroisoindol-2-yl)-piperidine-2,6-dione having the formula: 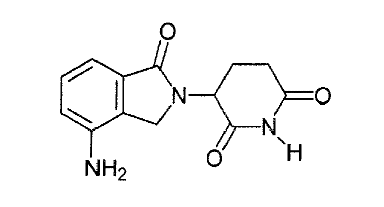 or a pharmaceutically acceptable salt, solvate or stereoisomer thereof, for use in a method for treating scleroderma.

2. The compound for use of claim 1, wherein the compound is enantiomerically pure.

3. The compound for use of claim 1, wherein the method comprises administration of a second active agent.

4. The compound for use of claim 3, wherein the second active agent is a keratolytic, retinoid, anti-inflammatory agent, immunosuppressive agent, herbal product, antibiotic, collagen, botulinum toxin, interferon, or immunomodulatory agent.

5. The compound for use of claim 3 or 4, wherein the second active agent is Xanthium Relieve Surface, 5-fluorouracil, masoprocol, trichloroacetic acid, salicylic acid, lactic acid, ammonium lactate, urea, isotretinoin, transretinoic acid, α-hydroxy acid, triamcinolone acetonide, collagen, botulinum toxin, or interferon.

6. The compound for use of claim 1 or 2, wherein the method comprises administration before, during or after photodynamic therapy or surgery.

7. The compound for use of any of claims 1 to 6, wherein the method comprises oral administration.

8. The compound for use of claim 7, wherein the compound is formulated as a capsule or tablet.

9. The compound for use of claims 1 to 8, wherein the method comprises cyclical administration.

10. The compound for use of any one of claims 1 to 9, wherein the method comprises administration in a single or divided daily doses in an amount of from about 0.10 to about 150 mg/day.

11. The compound for use of any one of claims 1 to 10, wherein the method comprises administration in an amount of from about 5 to about 25 mg per day or from about 25 to about 50 mg every other day.

12. The compound for use of any one of claims 1 to 11, wherein the compound is formulated as a dosage form comprising the compound in an amount of 5, 10, 25 or 50 mg.

13. The compound for use of any one of claims 1 to 9, wherein the compound is formulated as a dosage form comprising the compound in an amount of 0.1, 1, 2, 5, 7.5, 10, 12.5, 15, 17.5, 20, 25, 50, 100, 150 or 200 mg.
